# EUROPEAN PATENT APPLICATION

(11) **EP 1 584 335 A2**
(43) Date of publication of application: **12.10.2005**
(21) Application number: 04021315.9
(22) Date of filing: 08.09.2004
(51) Int. Cl.: A61K 45/06, A61P 25/04, A61P 25/06, A61P 29/00, A61P 13/00

(54) **Active substance combination comprising a carbinol composition and an opioid**

(30) Priority: 05.04.2004 ES 200400836; 27.08.2004 ES 200402102
(71) Applicant: LABORATORIOS DEL DR. ESTEVE, S.A., 08041 Barcelona (ES)
(72) Inventor: Buschmann, Helmut Heinrich, Barcelona (ES); Bonifacio, Gutierrez Silva, 08019 Barcelona (ES); Holenz, Joerg, 08012 Barcelona (ES); Farre Gomis, Antonio, 08037 Barcelona (ES)
(74) Representative: Davila Baz, Angel

(57) **Abstract**

The present invention relates to an active substance combination comprising at least one substituted carbinol compound and at least one opioid, a medicament comprising said active substance combination, a pharmaceutical formulation comprising said active substance combination and the use of said active substance combination for the manufacture of a medicament.

## Description

The present invention relates to an active substance combination comprising at least one substituted carbinol compound and at least one opioid, a medicament comprising said active substance combination, a pharmaceutical formulation comprising said active substance combination and the use of said active substance combination for the manufacture of a medicament.

Opioids such as morphine, which belong to the class of centrally acting analgesics, are key compounds for the treatment of moderate to very severe pain. However, in addition to their desired analgesic properties, these opioid analgesics show a multifaceted spectrum of undesired side effects, when administered to the patient in need of treatment, ranging from unpleasant effects such as emesis, inhibition of gastrointestinal function, sedation or dizziness to severe, often life-threatening effects such as respiratory depression. Further problems associated with the administration of opioids are the development of tolerance, the risk of addiction as well as the illicit use of such substances.

It was therefore an object of the present invention to provide a medicament with analgesic properties suitable for the treatment of moderate to very severe pain, which preferably does not show the undesired side effects of opioids, or at least less frequent and/or to a lesser extent.

It has now surprisingly been found that the pharmacological efficacy of opioids is enhanced by their administration in combination with one or more substituted carbinol compounds of general formula I given below. Consequently, the dose of the opioid analgesic may be reduced and fewer, less pronounced to none undesired side effects occur.

Thus, in one of its aspects the present invention relates to an active substance combination comprising
(A) at least one substituted carbinol compound of general formula I, 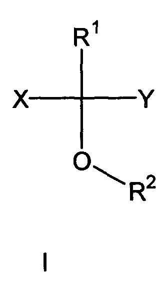 wherein
   R¹ represents a hydrogen atom, a linear or branched alkyl radical, a linear or branched alkenyl radical, an optionally at least mono-substituted cycloaliphatic radical, which may contain at least one nitrogen atom as ring member, or a phenyl radical,
   R² represents a hydrogen atom, an optionally at least one nitrogen atom as ring member containing cycloaliphatic radical, which may be at least mono-substituted by a linear or branched alkyl radical and/or which may be bound via a linear or branched alkylene group, a NR³R⁴-moiety, which is bound via a linear or branched alkylene group, or a NR⁵R⁶-moiety, which is bound via a linear or branched alkylene group,
   R³ and R⁴, identical or different, represent a linear or branched alkyl radical or an unsubstituted benzyl radical,
   R⁵ and R⁶ together with the bridging nitrogen atom represent a saturated, unsubstituted, optionally at least one further heteroatom as ring member containing heterocyclic radical,
   X represents an optionally at least mono-substituted phenyl radical or an optionally at least mono-substituted thienyl radical, wherein in each case the substituents are selected from the group consisting of a linear or branched alkyl radical, a linear or branched alkoxy group, a linear or branched alkyl radical, which is at least partially halogenated or a halogen atom,
   Y represents a heteroaryl radical, which contains one or more nitrogen atoms as ring members and which is unsubstituted or at least mono-substituted by one or more substitutents independently from one another selected from the group consisting of a halogen atom, a linear or branched alkyl radical, an unsubstituted benzyl radical, a ciano group bound via a linear or branched C₁₋₄-alkylene group, a carboxy group bound via a linear or branched C₁₋₄-alkylene group, a methoxy carbonyl group bound via a linear or branched C₁₋₄-alkylene group, a hydroxy group bound via a linear or branched C₁₋₄-alkylene group, an amino group bound via a linear or branched C₁₋₄-alkylene group, a (C₁₋₄) dialkylamino group bound via a linear or branched C₁₋₄-alkylene group and a cycloaliphatic radical, which contains one or more nitrogen atoms as ring members and which is bound via a linear or branched C₁₋₄-alkylene group, or Y represents an unsubstituted heteroaryl radical, which contains two nitrogen atoms as ring members and which is condensed with (annellated to) a saturated, one methyl-substituted nitrogen atom as ring member containing cycloaliphatic group,
   optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt, preferably a corresponding physiologically acceptable salt thereof, or a corresponding solvate, and
(B) at least one opioid.

Preferably the active substance combination according to the present invention comprises one or more substituted carbinol compounds of general formula I given above, wherein R¹ represents a hydrogen atom, a linear or branched C₁₋₄ alkyl radical, a linear or branched C₂₋₄ alkenyl radical, a 5-or 6-membered cycloaliphatic radical, which may contain at least one nitrogen atom as ring member and/or which may be at least mono-substituted by a linear or branched C₁₋₄ alkyl radical, or a phenyl radical, preferably a hydrogen atom, a linear or branched C₁₋₄ alkyl radical, a vinyl group, a cyclohexyl radical, an N-Methyl-piperidyl radical or a phenyl radical, and the other substituents R²-R⁶, X and Y have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

Also preferred the active substance combination according to the present invention comprises one or more substituted carbinol compounds of general formula I given above, wherein R² represents a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound vi a linear or branched C₁₋₄-alkyl radical, a NR³R⁴-moiety, which is bound via a linear or branched C₁₋₄ alkylene group, or a NR⁵R⁶-moiety, which is bound via a linear or branched C₁₋₄ alkylene group, prferably a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound vi a linear or branched C₁₋₄-alkyl radical, a NR³R⁴-moiety, which is bound via a linear or branched C₁₋₄ alkylene group, or a NR⁵R⁶-moiety, which is bound via a linear or branched C₁₋₄ alkylene group, and the remaining substituents R¹, R³-R⁶, X and Y have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

In another preferred embodiment of the present invention the inventive active substance combination comprises one or more substituted carbinol compounds of general formula I given above, wherein R³ and R⁴, identical or different, independently from one another represent a linear or branched C₁₋₄ alkyl radical or an unsubstituted benzyl radical, preferably a linear or branched C₁₋₄ alkyl radical, and the remaining substituents R¹, R², R⁵, R⁶, X and Y have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

Also preferred the active substance combination according to the present invention comprises one or more substituted carbinol compounds of general formula I given above, wherein R⁵ and R⁶ together with the bridging nitrogen atom represent a saturated, unsubstituted, optionally at least one oxygen atom as ring member containing, 5- or 6-membered heterocyclic radical, and the remaining substituents R¹-R⁴, X and Y have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

Also preferred the active substance combination according to the present invention comprises one or more substituted carbinol compounds of general formula I given above, wherein X represents an optionally at least mono-substituted phenyl radical or an optionally at least mono-substituted thienyl radical, wherein in each case the substituents are independently selected from the group consisting of a linear or branched C₁₋₄ alkyl radical, a linear or branched C₁₋₄ alkoxy radical, a linear or branched C₁₋₄ alkyl radical, which is at least partially fluorinated, a fluorine atom, a chlorine atom and a bromine atom, preferably an optionally at least mono-substituted phenyl radical or an optionally at least mono-substituted thienyl radical, wherein in each case the substituents are independently selected from the group consisting of a methyl radical, a methoxy radical, a trifluoromethyl radical, a fluorine atom, a chlorine atom and a bromine atom, and the remaining substituents R¹-R⁶ and Y have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

Also preferred the active substance combination according to the present invention comprises one or more substituted carbinol compounds of general formula I, wherein Y represents an azole radical selected from the group consisting of
a) a pyrazole of the general formula (a): 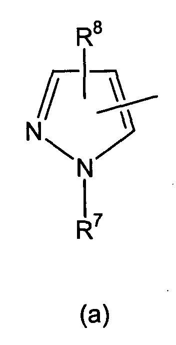 in which R⁷ represents a linear or branched C₁₋₁₂ alkyl radical, a benzyl radical or a radical of the type: 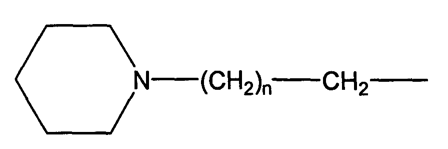 in which n = 1 or 2, and
   R⁸ represents a hydrogen atom, a methyl radical or a halogen atom, preferably a hydrogen atom, a methyl radical, a bromine atom or a chlorine atom,
b) an imidazole of the general formula 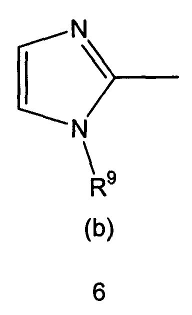 in which R⁹ represents a hydrogen atom, a C₁₋₁₂ alkyl radical, a benzyl radical or a radical of the general formula (b1):

   R¹⁰-(CH₂)ₙ- (b1)

   in which n is 2, 3 or 4 and R¹⁰ represents a piperidinyl radical, a phenyl radical, a cyano group, a hydroxyl radical, a carboxy radical, an amino group, a dimethylamino group or a methyl ester group,
   and
   an imidazole of the following formula: 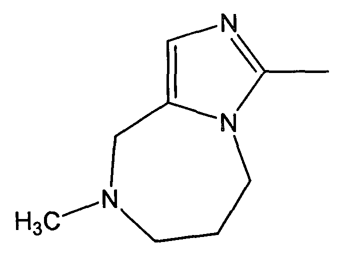 and the remaining substituents R¹-R⁶ and X have the meaning given above, optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

In a particularly preferred embodiment of the present invention the inventive active substance combination comprises one or more substituted carbinol compounds of general formula I 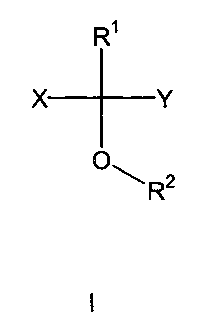 wherein
R¹ represents a hydrogen atom, a methyl radical, an ethyl radical, an n-propyl radical, an iso-propyl radical, a sec-butyl radical, a tert-butyl radical, an n-butyl radical, a vinyl radical, a cyclohexyl radical, an N-methyl-piperidinyl group, or a phenyl group,
R² represents a hydrogen atom, a dimethylaminoethyl group, a pyrrolidinylethyl group, a piperidinylethyl group, a methyl-benzyl-aminoethyl group, a morpholinylethyl group, a diisopropylaminoethyl group, a dimethylaminopropyl group, a piperidinylpropyl group, a pyrrolidinylpropyl group, a morpholinylpropyl group, an N-methyl-2-piperidyl group, an N-ethyl-2-piperidyl group, an N-propyl-2-piperidyl group, an N-methyl-2-pyrrolidinyl group, an N-ethyl-2-pyrrolidinyl group, an N-propyl-2-pyrrolidinyl group, or a 2-dimethylaminoethyl-1-methyl group,
X represents a phenyl radical, a 2-methyl-phenyl radical, a 3-methyl-phenyl radical, a 4-methyl phenyl radical, a 2-chloro-phenyl radical, a 3-chloro-phenyl radical, a 4-chloro-phenyl radical, a 2-fluoro-phenyl radical, a 3-fluoro-phenyl radical, a 4-fluoro-phenyl radical, a 2-trifluoromethyl-phenyl radical, a 3-trifluoromethyl-phenyl radical, a 4-trifluoromethyl-phenyl radical, a 2-methoxy-phenyl radical, a 3-methoxy-phenyl radical, a 4-methoxy-phenyl radical, a 3,4,5-tris-methoxy-phenyl radical, a 3,4-dichloro-phenyl radical, a 2,4-dichloro-phenylradical, a thien-2-yl radical, a thien-3-yl radical, a 3-methyl-thien-2-yl radical, a 5-methyl-thien-2-yl-radical, a 5-bromo-thien-2-yl radical or a 4-bromo-thien-2-yl-radical,
Y represents an azole radical selected from the group consisting of
a) a pyrazole of the general formula (a): 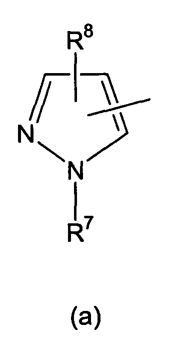 in which
   R⁷ represents a methyl radical, an ethyl radical, an n-propyl radical, an iso-propyl radical, an n-butyl radical, a sec-butyl radical or a tert-butyl radical,
   R⁸ represents a hydrogen atom, a methyl radical, a bromine atom or a chlorine atom,
b) an imidazole of the general formula 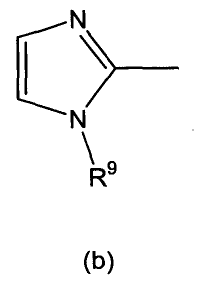 in which R⁹ represents a hydrogen atom, a methyl radical, an ethyl radical, an n-propyl radical, an iso-butyl radical, an n-butyl radical, a sec-butyl radical a tert-butyl radical, an n-pentyl radical, an n-hexyl radical, an n-heptyl radical, an n-octyl radical, an n-nonyl radical, an n-decyl radical, an n-undecyl radical an n-dodecyl radical, a benzyl radical, or a radical of the general formula (b1):

   R¹⁰-(CH₂)ₙ- (b1)

   in which n is 2, 3 or 4 and R¹⁰ represents a piperidinyl radical, a phenyl radical, a cyano group, a hydroxyl radical, a carboxy radical, an amino group, a dimethylamino group, or a methyl ester group,
   and
(c) an imidazole of the following formula:

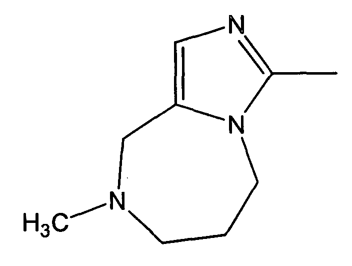

In a most particularly preferred embodiment of the present invention the inventive active substance combination comprises one or more substituted carbinol compounds selected from the group consisting of:
[1] 2-{α-[2-(dimethylamino)ethoxy]benzyl}-1-methyl-1*H*-imidazole,
[2] 2-{4-chloro-α-[2-(dimethylamino)ethoxy]-α-methylbenzyl}-1-methyl-1*H*-imidazole,
[3] 2-{4-chloro-α-[2-(dimethylamino)ethoxy]benzyl}-1-methyl-1*H*-imidazole,
[4] 2-{3-chloro-α-[2-(dimethylamino)ethoxy]benzyl}-1-methyl-1*H*-imidazole
[5] 2-{4-chloro-α-[2-(dimethylamino)ethoxy]-α-methylbenzyl}-1-methyl-1*H*-imidazole,
[6] 2-{4-fluoro-α-[2-(dimethylamino)ethoxy]-α-methylbenzyl}-1-methyl-1*H*-imidazole,
[7] 2-{α-[2-(dimethylamino)ethoxy]-α-methyl-3-(trifluoromethyl)benzyl}-1-methyl-1*H*-imidazole,
[8] 2-{3-chloro-α-[2-(dimethylamino)ethoxy]-α-methylbenzyl}-1-methyl-1*H*-imidazole,
[9] 2-{3-chloro-α-[2-(dimethylamino)ethoxy]-α-propylbenzyl}-1-methyl-1*H*-imidazole,
[10] 1-butyl-2-{4-chloro-α-[2-(dimethylamino)ethoxy]-α-methylbenzyl}-1*H*-imidazole,
[11] 2-{α-[2-(dimethylamino)ethoxy]-α-methyl-4-methoxybenzyl}-1-methyl-1*H*-imidazole,
[12] 2-{3-chloro-α-methyl-α-[2-(N-pyrrolidinyl)ethoxy]benzyl}-1-methyl-1*H*-imidazole,
[13] 2-{α-[2-(dimethylamino)ethoxy]-α-propyl-3,4,5-trimethoxybenzyl}-1-dodecyl-1*H*-imidazole,
[14] 1-butyl-2-{α-[2-(dimethylamino)ethoxy]-4-(trifluoromethyl)benzyl}-1*H*-imidazole,
[15] 1-methyl-2-{α-methyl-α-[2-(N-piperidyl)ethoxy]-3-(trifluoromethyl)benzyl}-1*H*-imidazole,
[16] 2-{α-cyclohexyl-3,4-dichloro-α-[2-(dimethylamino)ethoxy]benzyl}-1-methyl-1*H*-imidazole,
[17] 2-{3,4-dichloro-α-[2-(dimethylamino)ethoxy]-α-propylbenzyl}-1-methyl-1*H*-imidazole,
[18] 2-{3,4-dichloro-α-[2-(dimethylamino)ethoxy]-α-methylbenzyl}-1-methyl-1*H*-imidazole,
[19] 2-{3,4-dichloro-α-[2-(dimethylamino)ethoxy]benzyl}-1-methyl-1*H*-imidazole,
[20] 2-{4-chloro-α-[2-(dimethylamino)ethoxy]-α-methylbenzyl}-1-[2-(N-piperidyl)ethyl]-1*H*-imidazole,
[21] 2-{4-chloro-α-[2-(dimethylamino)ethoxy]-α-methylbenzyl}-1-[2-(N-piperidyl)propyl]-1*H*-imidazole,
[22] 1-(3-cyanopropyl)-2-{4-chloro-α-[2-(dimethylamino)ethoxy]benzyl}-1*H*-imidazole,
[23] 2-{4-chloro-α-[2-(dimethylamino)ethoxy]-α-(N-methyl-4-piperidyl)benzyl}-1-methyl-1*H*-imidazole,
[24] 1-benzyl-2-{α-[2-(N-benzyl-N-methylamino)ethoxy]-4-chlorobenzyl}-1*H*-imidazole,
[25] 2-{4-chloro-α-[2-(dimethylamino)ethoxy]-α-methylbenzyl}-7-methyl-6,7,8,9-tetrahydro-1*H*-imidazole[1,5-a][1,4]diazepine,
[26] 2-{4-chloro-α-[2-(dimethylamino)ethoxy]benzyl}-7-methyl-6,7,8,9-tetrahydro-1*H*-imidazole[1,5-a][1,4]diazepine,
[27] 1-butyl-5-{α-[2-(dimethylamino)ethoxy]benzyl}-1*H*-pyrazole,
[28] 5-{α-(4-chlorophenyl)-α-[2-(dimethylamino)ethoxy]benzyl}-1-methyl-1*H*-yrazole,
[29] 1-butyl-5-{α-[2-(dimethylamino)ethoxy]-3,4,5-trimethoxybenzyl}-1*H*-pyrazole,
[30] 1-butyl-5-{4-chloro-α-[2-(dimethylamino)ethoxy]-α-methylbenzyl}-1*H*-pyrazole,
[31] 5-{α-[2-(dimethylamino)ethoxy]benzyl}-1-methyl-1*H*-pyrazole,
[32] 5-{α-[2-(dimethylamino)ethoxy]-α-methylbenzyl}-1-methyl-1*H*-pyrazole,
[33] 5-{α-[2-(dimethylamino)ethoxy]-3,4,5-trimethoxybenzyl}-1-methyl-1*H*-pyrazole,
[34] 1-methyl-5-{α-[2-(N-pyrrolidinyl)ethoxy]benzyl}-1*H*-pyrazole,
[35] 1-methyl-5-{α-[2-(N-morpholinyl)ethoxy]benzyl}-1*H*-pyrazole,
[36] 5-{α-[2-(dimethylamino)ethoxy]-α-methyl-3,4,5-trimethoxybenzyl}-1-methyl-1*H*-pyrazole,
[37] 4-bromo-5-{α-[2-(dimethylamino)ethoxy]benzyl}-1-methyl-1*H*-pyrazole,
[38] 1,3-dimethyl-5-{α-[2-(dimethylamino)ethoxy]-α-methylbenzyl}-1*H*-pyrazole,
[39] 1,3-dimethyl-5-{α-[2-(dimethylamino)ethoxy]benzyl}-1*H*-pyrazole,
[40] 5-{α-[2-(dimethylamino)ethoxy]-2-methylbenzyl}-1-methyl-1*H*-pyrazole,
[41] 4-chloro-5-{4-chloro-α-[2-(dimethylamino)ethoxy]benzyl}-1-methyl-1*H*-yrazole,
[42] 5-{4-chloro-α-[2-(dimethylamino)ethoxy]benzyl}-1-methyl-1*H*-pyrazole,
[43] 5-{3-chloro-α-[2-(dimethylamino)ethoxy]benzyl}-1-methyl-1*H*-pyrazole,
[44] 5-{α-[2-(dimethylamino)ethoxy]-4-methylbenzyl}-1-methyl-1*H*-pyrazole,
[45] 5-{2-chloro-α-[2-(dimethylamino)ethoxy]benzyl}-1-methyl-1*H*-pyrazole,
[46] 1-methyl-5-{α-[2-(N-piperidyl)ethoxy]benzyl}-1*H*-pyrazole,
[47] 1-methyl-5-{α-[2-(N-propyl-2-piperidyl)ethoxy]benzyl}-1*H*-pyrazole,
[48] 5-{α-[2-(N-ethyl-2-piperidyl)ethoxy]benzyl}-1-methyl-1*H*-pyrazole,
[49] 1-methyl-5-{α-[2-(N-methyl-2-pyrrolidinyl)ethoxy]benzyl}-1*H*-pyrazole,
[50] 5-{α-[2-(diisopropylamino)ethoxy]benzyl}-1-methyl-1*H*-pyrazole,
[51] 1-methyl-5-{α-[2-(N-methyl-2-piperidyl)ethoxy]benzyl}-1*H*-pyrazole,
[52] 2-{4-chloro-α-[3-(dimethylamino)propoxy]-α-methylbenzyl}-1-methyl-1*H*-imidazole,
[53] 2-{3-chloro-α-[3-(dimethylamino)propoxy]benzyl}-1-methyl-1*H*-imidazole
[54] 2-{4-chloro-α-[3-(dimethylamino)propoxy]-α-ethylbenzyl}-1-methyl-1*H*-imidazole,
[55] 2-{α-butyl-3-chloro-α-[3-(dimethylamino)propoxy]benzyl}-1-methyl-1*H*-imidazole,
[56] 2-{α-cyclohexyl-4-chloro-α-[3-(dimethylamino)propoxy]benzyl}-1-methyl-1*H*-imidazole,
[57] 2-{α-[3-(dimethy)amino)propoxy]-4-fluoro-α-methy)benzyl}-1-methyl-1*H*-imidazole,
[58] 2-{α-[3-(dimethylamino)propoxy]-α-methyl-3-(trifluoromethyl)benzyl}-1-methyl-1*H*-imidazole,
[59] 2-{2-chloro-α-[3-(dimethylamino)propoxy]-α-methylbenzyl}-1-methyl-1*H*-imidazole,
[60] 2-{3-chloro-α-[3-(dimethylamino)propoxy]-α-methylbenzyl}-1-methyl-1*H*-imidazole,
[61] 2-{α-[3-(dimethylamino)propoxy]-α-methyl-3,4,5-trimethoxybenzyl}-1-methyl-1*H*-imidazole,
[62] 2-{α-[3-(dimethy)amino)propoxy]-α-methyl-4-methoxybenzyl}-1-methyl-1*H*-imidazole,
[63] 2-{4-chloro-α-[3-(dimethylamino)propoxy]benzyl}-1-methyl-1*H*-imidazole,
[64] 2-{α-[3-(dimethylamino)propoxy]-3,4,5-trimethoxybenzyl}-1-methyl-1*H*-imidazole,
[65] 2-{α-[3-(dimethylamino)propoxy]-α-methyl-4-(trifluoromethyl)benzyl}-1-methyl-1*H*-imidazole,
[66] 2-{α-[3-(dimethylamino)propoxy]-3-(trifluoromethyl)benzyl}-1-methyl-1*H*-imidazole,
[67] 2-{α-[3-(dimethylamino)propoxy]-4-(trifluoromethyl)benzyl}-1-methyl-1*H*-imidazole,
[68] 2-{α-[3-(dimethylamino)propoxy]-4-methoxybenzyl}-1-methyl-1*H*-imidazole,
[69] 2-{α-butyl-α-[3-(dimethylamino)propoxy]-3-(trifluoromethyl)benzyl}-1-methyl-1*H*-imidazole,
[70] 1-butyl-2-{4-chloro-α-[3-(dimethylamino)propoxy]-α-methylbenzyl}-1*H*-imidazole,
[71] 1-butyl-2-{α-butyl-α-[3-(dimethylamino)propoxy]-3,4,5-trimethoxybenzyl}-1*H*-imidazole,
[72] 1-butyl-2-{α-butyl-2-chloro-α-[3-(dimethylamino)propoxy]benzyl}-1*H*-imidazole,
[73] 1-butyl-2-{α-butyl-2,4-dichloro-α-[3-(dimethylamino)propoxy]benzyl}-1*H*-imidazole,
[74] 1-butyl-2-{α-[3-(dimethylamino)propoxy]-4-(trifluoromethyl)benzyl}-1*H*-imidazole,
[75] 2-{4-chloro-α-[3-(N-piperidyl)propoxy]benzyl}-1-methyl-1*H*-imidazote,
[76] 1-methyl-2-{α-methyl-α-[3-(N-piperidyl)propoxy]-4-(trifluoromethyl)benzyl}-1*H*-imidazole,
[77] 2-{α-butyl-2-chloro-α-[3-(dimethylamino)propoxy]benzyl}-1-methyl-1*H*-imidazole,
[78] 2-{α-butyl-3,4-dichloro-α-[3-(dimethylamino)propoxy]benzyl}-1-methyl-1*H*-imidazole,
[79] 2-{3,4-dichloro-α-[3-(dimethylamino)propoxy]-α-methylbenzyl}-1-methyl-1*H*-imidazole,
[80] 2-{3,4-dichloro-α-[3-(dimethylamino)propoxy]benzyl}-1-methyl-1*H*-imidazole,
[81] 2-{α-cyclohexyl-3,4-dichloro-α-[3-(dimethylamino)propoxy]benzyl}-1-methyl-1*H*-imidazole,
[82] 2-{4-chloro-α-[3-(dimethylamino)propoxy]-α-methylbenzyl}-α-[2-(N-piperidyl) ethyl]-1*H*-imidazole,
[83] 2-{4-chloro-α-[3-(dimethylamino)propoxy]-α-methylbenzyl}-1-[2-(N-piperidyl) propyl]-1*H*-imidazole,
[84] 2-{4-chloro-α-[3-(dimethylamino)propoxy]-α-(N-methyl-4-piperidyl)benzyl}-1-methyl-1 *H*-imidazole,
[85] 1-butyl-5-{α-[3-(dimethylamino)propoxy]benzyl}-1*H*-pyrazole,
[86] 1-butyl-5-(4-chloro-α-[3-(dimethylamino)propoxy]-α-methylbenzyl)-1*H*-pyrazole,
[87] 5-{α-[3-(dimethylamino)propoxy]benzyl}-1-methyl-1*H*-pyrazole,
[88] 5-{α-[3-(dimethylamino)propoxy]-α-methylbenzyl}-1-methyl-1*H*-pyrazole,
[89] 1,3-dimethyl-5-{α-[3-(dimethylamino)propoxy]-α-methylbenzyl}-1*H*-pyrazole,
[90] 1,3-dimethyl-5-{α-[3-(dimethylamino)propoxy]benzyl}-1*H*-pyrazole,
[91] 5-{α-[3-(dimethylamino)propoxy]-2-methylbenzyl}-1-methyl-1*H*-pyrazole,
[92] 5-chloro-5-{4-chloro-α-[3-(dimethylamino)propoxy]benzyl}-1-methyl-1*H*-pyrazole,
[93] 1-methyl-5-{α-[3-(N-piperidyl)propoxy]benzyl}-1*H*-pyrazole,
[94] 1-methyl-5-{α-[3-(N-pyrrolidinyl)propoxy]benzyl}-1*H*-pyrazole,
[95] 4-{4-chloro-α-[2-(dimethylamino)ethoxy]benzyl}-1-methyl-1*H*-pyrazole,
[96] 4-{4-chloro-α-[2-(dimethylamino)ethoxy]-α-methylbenzyl}-1-methyl-1*H*-pyrazole,
[97] 4-{4-chloro-α-[2-(N-propyl-2-piperidyl)ethoxy]benzyl}-1-methyl-1*H*-pyrazole,
[98] 4-{4-chloro-α-[2-(N-methyl-2-piperidyl)ethoxy]benzyl}-1-methyl-1*H*-pyrazole,
[99] 4-{4-chloro-α-[2-(N-ethyl-2-piperidyl)ethoxy]benzyl}-1-methyl-1*H*-pyrazole,
[100] 4-{4-chloro-α-[2-(diisopropylamino)ethoxy]benzyl}-1-methyl-1*H*-pyrazole,
[101] 4-{4-chloro-α-[2-(N-methyl-2-pyrrolidinyl)ethoxy]benzyl}-1-methyl-1*H*-pyrazole,
[102] 4-{α-[3-(dimethylamino)propoxy]benzyl}-1-methyl-1*H*-pyrazole,
[103] 4-{4-chloro-α-[3-(N-morpholinyl)propoxy]benzyl}-1-methyl-1*H*-pyrazole,
[104] 4-{4-chloro-α-[3-(N-pyrrolidinyl)propoxy]benzyl}-1-methyl-1*H*-pyrazole,
[105] 2-(α-hydroxybenzyl)-1*H*-imidazole,
[106] 2-(4-chloro-α-hydroxybenzyl)-1*H*-imidazole,
[107] 2-(4-chloro-α-hydroxybenzyl)-1-methyl-1*H*-imidazole,
[108] 2-(3-chloro-α-hydroxybenzyl)-1-methyl-1*H*-imidazole,
[109] 2-(4-fluoro-α-hydroxybenzyl)-1-methyl-1*H*-imidazole,
[110] 2-[α-hydroxy-3-(trifluoromethyl)benzyl]-1-methyl-1*H*-imidazole,
[111] 2-[α-hydroxy-4-(trifluoromethyl)benzyl]-1-methyl-1*H*-imidazole,
[112] 2-(α-hydroxy-3,4,5-trimethoxybenzyl)-1-methyl-1*H*-imidazole,
[113] 2-(3,4-dichloro-α-hydroxybenzyl)-1-methyl-1*H*-imidazole,
[114] 1-butyl-2-[α-hydroxy-4-(trifluoromethyl)benzyl]-1*H*-imidazole,
[115] 1-butyl-2-(3,4-dichloro-α-hydroxybenzyl)-1*H*-imidazole,
[116] 1-butyl-2-(4-chloro-α-hydroxybenzyl)-1*H*-imidazole,
[117] 1-butyl-2-(α-hydroxy-3,4,5-trimethoxybenzyl)-1*H*-imidazole,
[118] 1-dodecyl-2-(α-hydroxy-3,4,5-trimethoxybenzyl)-1*H*-imidazole,
[119] 2-(α-butyl-3-chloro-α-hydroxybenzyl)-1-methyl-1*H*-imidazole,
[120] 2-(3-chloro-α-hydroxy-α-methylbenzyl)-1-methyl-1*H*-imidazole,
[121] 2-(4-chloro-α-hydroxy-α-methylbenzyl)-1-methyl-1*H*-imidazole,
[122] 2-[4-chloro-α-hydroxy-α-(N-methyl-4-piperidyl)benzyl]-1-methyl-1*H*-imidazole,
[123] 2-(4-chloro-α-ethyl-α-hydroxybenzyl)-1-methyl-1*H*-imidazole,
[124] 2-(α-butyl-4-chloro-α-hydroxybenzyl)-1-methyl-1*H*-imidazole,
[125] 2-(α-cyclohexyl-4-chloro-α-hydroxybenzyl)-1-methyl-1*H*-imidazole,
[126] 2-(2-chloro-α-hydroxy-α-methylbenzyl)-1-methyl-1*H*-imidazole,
[127] 2-(α-butyl-2-chloro-α-hydroxybenzyl)-1-methyl-1*H*-imidazole,
[128] 2-[α-hydroxy-α-methyl-3-(trifluoromethyl)benzyl]-1-methyl-1*H*-imidazole,
[129] 2-[α-butyl-α-hydroxy-3-(trifluoromethyl)benzyl]-1-methyl-1*H*-imidazole
[130] 2-[α-cyclohexyl-α-hydroxy-3-(trifluoromethyl)benzyl]-1-methyl-1*H*-imidazole,
[131] 2-[α-hydroxy-α-methyl-4-(trifluoromethyl)benzyl]-1-methyl-1*H*-imidazole,
[132] 2-(4-fluoro-α-hydroxy-α-methylbenzyl)-1-methyl-1*H*-imidazole,
[133] 2-(α-hydroxy-α-methyl-4-methoxybenzyl)-1-methyl-1*H*-imidazole,
[134] 2-(3,4-dichloro-α-hydroxy-α-methylbenzyl)-1-methyl-1*H*-imidazole,
[135] 2-(α-butyl-3,4-dichloro-α-hydroxybenzyl)-1-methyl-1*H*-imidazole,
[136] 2-(α-cyclohexyl-3,4-dichloro-α-hydroxybenzyl)-1-methyl-1*H*-imidazole,
[137] 2-(α -hydroxy-α-methyl-3,4,5-trimethoxybenzyl)-1-methyl-1*H*-imidazole,
[138] 1-butyl-2-(4-chloro-α-hydroxy-α-methylbenzyl)-1*H*-imidazole,
[139] 1-butyl-2-(α-butyl-4-chloro-α-hydroxybenzyl]-1*H*-imidazole,
[140] 1-butyl-2-[4-chloro-α-hydroxy-α-(N-methyl-4-piperidyl)benzyl]-1*H*-imidazole,
[141] 1-butyl-2-(α-butyl-α-hydroxy-3,4,5-trimethoxybenzyl)-1*H*-imidazole,
[142] 1-butyl-2-(α-butyl-2-chloro-α-hydroxybenzyl)-1*H*-imidazole,
[143] 1-butyl-2-[α-ethyl-α-hydroxy-3-(trifluoromethyl)benzyl]-1*H*-imidazole,
[144] 1-butyl-2-(α-butyl-2,4-dichloro-α-hydroxybenzyl)-1*H*-imidazole,
[145] 2-(4-chloro-α-hydroxy-α-methylbenzyl)-1-[2-(N-piperidyl)ethyl]-1*H*-imidazole,
[146] 2-(4-chloro-α-hydroxy-α-methylbenzyl)-1-(3-dimethylaminopropyl)-1*H*-imidazole,
[147] 2-(α-butyl-α -hydroxy-3,4,5-trimethoxybenzyl)-1-dodecyl-1*H*-imidazole,
[148] 1-benzyl-2-[α-butyl-α-hydroxy-3-(trifluoromethyl)benzyl]-1*H*-imidazole,
[149] 1-benzyl-2-(4-chloro-α-hydroxy-α-methylbenzyl)-1*H*-imidazole,
[150] 1-(2-cyanoethyl)-2-(4-chloro-α-hydroxybenzyl)-1*H*-imidazole,
[151] 1-(3-aminopropyl)-2-(4-chloro-α-hydroxybenzyl)-1*H*-imidazole,
[152] 3-[2-(3-chloro-α-hydroxybenzyl)-1*H*-imidazole-1-yl]propanoic acid
[153] 2-(4-chloro-α-hydroxybenzyl)-1-(3-hydroxypropyl)-1*H*-imidazole,
[154] 3-[2-(3-chloro-α-hydroxybenzyl)-1*H*-imidazole-1-yl]methyl-propanoate
[155] 2-(α-hydroxybenzyl)-1-(3-hydroxypropyl)-1*H*-imidazole,
[156] 2-(α-hydroxy-4-methylbenzyl)-1-(3-hydroxypropyl)-1*H*-imidazole,
[157] 2-(α-hydroxy-4-methoxybenzyl)-1-(3-hydroxypropyl)-1*H*-imidazole,
[158] 2-(3,4-dichloro-α-hydroxybenzyl)-1-(3-hydroxypropyl)-1*H*-imidazole,
[159] 3-{2-(α-hydroxybenzyl)-1*H*-imidazole-1-yll}-methyl propanoate
[160] 2-(4-chloro-α-hydroxybenzyl)-1-(4-hydroxybutyl)-1*H*-imidazole,
[161] 1-(3-cyanopropyl)-2-(4-chloro-α-hydroxybenzyl)-1*H*-imidazole,
[162] 4-[2-(4-chloro-α-hydroxybenzyl)-1*H*-imidazole-1-yl]butanoic acid,
[163] 4-[2-(4-chloro-α-hydroxybenzyl)-1*H*-imidazole-1-yl]-methyl butanoate,
[164] 1-butyl-5-(α-hydroxybenzyl)-1*H*-pyrazole,
[165] 5-(4-chloro-α-hydroxybenzyl)-1-methyl-1*H*-pyrazole,
[166] 5-(α-hydroxy-3,4,5-trimethoxybenzyl)-1-methyl-1*H*-pyrazole,
[167] 1-butyl-5-(α-hydroxy-3,4,5-trimethoxybenzyl)-1*H*-pyrazole,
[168] 4-bromo-5-(α-hydroxybenzyl)-1-methyl-1*H*-pyrazole,
[169] 5-[α-(4-chlorophenyl)-α-hydroxybenzyl]-1-methyl-1*H*-pyrazole,
[170] 1-butyl-5-(4-chloro-α-hydroxy-α-methylbenzyl)-1*H*-pyrazole,
[171] 5-(α-hydroxy-α-methylbenzyl)-1-methyl-1*H*-pyrazole,
[172] 5-(α-hydroxy-α-methyl-3,4,5-trimethoxybenzyl)-1-methyl-1*H*-pyrazole,
[173] 1,3-dimethyl-5-(α-hydroxy-α-methylbenzyl)-1*H*-pyrazole,
[174] 1-butyl-5-(α-hydroxy-α-vinylbenzyl)-1*H*-pyrazole,
[175] 1-butyl-5-(4-chloro-α-hydroxy-α-vinylbenzyl)-1*H*-pyrazole,
[176] 4-chloro-5-(α-hydroxybenzyl)-1-methyl-1*H*-pyrazole,
[177] 5-(α-hydroxy-2-methylbenzyl)-1-methyl-1*H*-pyrazole,
[178] 5-(3-chloro-α-hydroxybenzyl)-1-methyl-1*H*-pyrazole,
[179] 5-(α-hydroxy-4-methylbenzyl)-1-methyl-1*H*-pyrazole,
[180] 5-(2-chloro-α-hydroxybenzyl)-1-methyl-1*H*-pyrazole,
[181] 5-(α-hydroxy-4-methoxybenzyl)-1-methyl-1*H*-pyrazole,
[182] 5-{α-[2-(dimethylamino)ethoxy]-2-thienylmethyl}-1-methyl-1*H*-pyrazole,
[183] 5-{α-[2-(dimethylamino)ethoxy]-2-thienylmethyl}-1-methyl-1*H*-pyrazole citrate,
[184] 5-{α-[2-(dimethylamino)ethoxy]-3-thienylmethyl}-1-methyl-1*H*-pyrazole,
[185] 2-{α-[2-(dimethylamino)ethoxy]-2-thienylmethyl}-1-methyl-1*H*-imidazole,
[186] 5-{α-[2-(dimethylamino)ethoxy]-3-methyl-2-thienylmethyl}-1-methyl-1*H*-pyrazole,
[187] 5-{α-[2-(dimethylamino)ethoxy]-5-methyl-2-thienylmethyl}-1-methyl-1*H*-pyrazole,
[188] 5-{5-bromo-α-[2-(dimethylamino)ethoxy]-2-thienylmethyl}-1-methyl-1*H*-pyrazole,
[189] 5-{4-bromo-α-[2-(dimethylamino)ethoxy]-2-thienylmethyl}-1-methyl-1*H*-pyrazole,
[190] 5-{α-[2-(dimethylamino)ethoxy]-α-methyl-2-thienylmethyl}-1-methyl-1*H*-pyrazole,
[191] 5-{α-[2-(dimethylamino)ethoxy]benzyl}-1-methyl-1*H*-pyrazole citrate
[192] (±)-5-{α-[2-(dimethy)amino)-1-(methyl)ethoxy]benzyl}-1-methyl-1*H*-pyrazole,
[193] (±)-5-{α-[2-(dimethylamino)-1-(methyl)ethoxy]benzyl}-1-methyl-1*H*-pyrazole,
[194] (+)-5-{α-[2-(dimethylamino)ethoxy]-2-thienylmethyl}-1-methyl-1*H*-pyrazole,
[195] (-)-5-{α-[2-(dimethylamino)ethoxy]-2-thienylmethyl}-1-methyl-1*H*-pyrazole,
[196] (+)-5-{α-[2-(dimethylamino)ethoxy]-2-thienylmethyl}-1-methyl-1*H*-pyrazole citrate,
[197] (-)-5-{α-[2-(dimethylamino)ethoxy]-2-thienylmethyl}-1-methyl-1*H*-pyrazole citrate,
[198] (+)-5-{α-[2-(dimethylamino)ethoxy]-2-thienylmethyl}-1-methyl-1*H*-pyrazole-D-ditoluyltartrat,
[199] (-)-5-{α-[2-(dimethylamino)ethoxy]-2-thienylmethyl}-1-methyl-1*H*-pyrazole D-ditoluyltartrat,
[200] (+)-5-{α-[2-(dimethylamino)ethoxy]benzyl}-1-methyl-1*H*-pyrazole citrate,
[201] (-)-5-{α-[2-(dimethylamino)ethoxy]benzyl}-1-methyl-1*H*-pyrazole citrate,
[202] 5-(α-hydroxy-2-thienylmethyl)-1-methyl-1*H*-pyrazole,
[203] 5-(α-hydroxy-3-methyl-2-thienylmethyl)-1-methyl-1*H*-pyrazole,
[204] 5-(α-hydroxy-5-methyl-2-thienylmethyl)-1-methyl-1*H*-pyrazole,
[205] 5-(5-bromo-α-hydroxy-2-thienylmethyl)-1-methyl-1*H*-pyrazole,
[206] 5-(4-bromo-α-hydroxy-2-thienylmethyl)-1-methyl-1*H*-pyrazole and
[207] 5-(α-hydroxy-α-methyl-2-thienylmethyl)-1-methyl-1*H*-pyrazole
as component (A).

The preparation of the substituted carbinol compounds of general formula I, their stereoisomers, corresponding salts and corresponding solvates may be accomplished by the reagents and methods described, for example, in EP 0289 380, US 5,017,596, WO99/52525 (US 6,410,582) and WO99/07684 (US 6,118,009). Methods for the optical resolution of said compounds, i.e. the preparation or separation of the respective stereoisomers are described, for example, in WO99/02500 (US 6,187,930) and WO97/20817 (US 5,849,931). The corresponding parts of these publications are hereby incorporated by reference and form part of the present disclosure.

Physiologically acceptable salts of the substituted carbinol compounds of general formula I given above may be obtained by conventional methods known to those skilled in the art. Preferred pharmaceutically acceptable salts of these substituted carbinol compounds of general formula I given above are the citrate salts or the ditoluyltartrate salts. Generally included are also addition salts of mineral acids or of organic acids such as oxalate, tartrate, citrate and hydroquinonesulfate. Additionally, the term "salt" is to be understood as including any form of an active compound of the inventive active substance combination in which this is present in ionic or charged form and is coupled with a corresponding counter-ion (a cation or anion) or is in solution. The term "salt" further comprises complexes of an active compound of the inventive active substance combination with other ions or molecules, in particular complexes, which are complexed via ionic interactions.

In the context of the present invention, the term "physiologically acceptable salt" is understood in particular as including a salt that is formed either with a physiologically tolerated acid, that is to say salts of the particular active compound with inorganic or organic acids which are physiologically tolerated - especially if used on humans and/or mammals - or with at least one, preferably inorganic, ion, preferably cation, which are physiologically tolerated, especially if used on humans and/or mammals. Examples of physiologically tolerated salts of particular acids are salts of hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid, citric acid, glutamic acid, 1,1-dioxo-1,2-dihydro-6-benzo[d]isothiazol-3-one (saccharin acid), monomethylsebacic acid, 5-oxo-proline, hexane-1-sulfonic acid, nicotinic acid, 2-,3- or 4-aminobenzoic acid, 2,4,6-trimethyl-benzoic acid, alpha-lipoic acid, acetylglycine, acetylsalicylic acid, hippuric acid and/or aspartic acid. Examples of physiologically tolerated salts of particular bases are salts of alkali and alkaline earth metals and/or with {NHₓR₄₋ₓ]⁺-ions, wherein x is 0, 1, 2, 3 or 4 and R represents a linear or branched C₁₋₄ alkyl radical.

With regard to the compounds of component A of the inventive active substance combination the salts that are preferred are salts of physiologically tolerated acids.

The salt, which is particularly preferred for the particular compound of component A is the citrate.

For the purposes of the present invention the term opioid includes substances having affinity for one or more of the opioid receptors such as the µ-opioid receptors, the δ-opioid receptors and/or the κ-opioid receptors. Preferred are opioids, which act as agonists or partial agonists on these receptors as well as mixed agonists/antagonists. Preferred are also compounds that act as antagonists, either if used alone or in combination with other compounds of component (B).

Suitable opioids according to component (B) of the inventive pharmacologically active substance combination as well as methods for their preparation are well known to those skilled in the art, e.g. from E. Friderichs, T. Christoph and H. Buschmann, "Analgesics and Antipyretics", Ullmann's Encyclopedia of Industrial Chemistry, Sixth Edition, Wiley-VCH Verlag GmbH, Weinheim 2000, pages 27-45. The opioid 14-Methoxymetopon is for example described in the publication of M.A. King et al., Eur. J. of Pharmacology, 459 (2003), 203-209. The respective descriptions are hereby incorporated by reference and form part of the present disclosure, especially as sources for the election of the opioids according to component B of the inventive active substance combination.

Preferably the inventive active substance combination comprises as component (B) at least one opoid with weak analgesic efficacy, which may preferably be selected from the group consisting of codeine, dextropropoxyphene, dihydrocodeine, diphenoxylate, ethylmorphine, loperamide, meptazinol, nalbuphine, pethidine, tilidine, tramadol, viminol and corresponding physiologically acceptable salts of these compounds.

Even though neither the active substance combination of the present invention nor any compound according to component A do give rise to concerns regarding addiction it may also be preferred to include either in addition to an active substance combination according to the present invention or as compound B an opioid antagonist like Naloxone or Naltrexone.

If the active substance combination of the present invention comprises as component (B) an opioid with weak analgesic efficacy, the molar ratio of component (B) to component (A) is preferably in the range of 1:1 to 1:20, preferably 1:1 to 1:10, more preferably 1:1 to 1:5.

Also preferably, the inventive active substance combination comprises one or more opioid analgesics with medium to strong analgesic efficacy, which may preferably be selected from the group consisting of alfentanil, buprenorphine, butorphanol, dextromoramide, dezocine, diacetylmorphine (heroine), etorphine, fentanyl, hydrocodone, hydromorphone, ketobemidone, levomethadone, levomethadyl acetate, levorphanol, morphine, nalorphine, oxycodone, oxymorphone, pentazocine, piritramide, remifentanil, sufentanil and corresponding physiologically acceptable salts thereof.

If the active substance combination of the present invention comprises as component (B) an opioid with medium to strong analgesic efficacy, the molar ratio of component (B) to component (A) is in the range of 1:1 to 1:400, preferably 1:1 to 1:200, more preferably 1:1 to 1:10, most preferably 1:1 to 1:5.

Physiologically acceptable salts of the opioid analgesics according to component (B) of the inventive active substance combination are also well known to those skilled in the art and may preferably be selected from the group consisting of hydrochloride, hydrobromide, sulfate, phosphate, tartrate, citrate and acetate. Generally included are addition salts of mineral acids or of organic acids such as oxalate, tartrate, citrate and hydroquinonesulfate. Additionally, the term "salt" is to be understood as including any form of an active compound of the inventive active substance combination in which this is present in ionic or charged form and is coupled with a corresponding counter-ion (a cation or anion) or is in solution. The term "salt" further comprises complexes of an active compound of the inventive active substance combination with other ions or molecules, in particular complexes, which are complexed via ionic interactions.

In the context of the present invention, the term "physiologically acceptable salt" is understood in particular as including a salt that is formed either with a physiologically tolerated acid, that is to say salts of the particular active compound with inorganic or organic acids which are physiologically tolerated - especially if used on humans and/or mammals - or with at least one, preferably inorganic, ion, preferably cation, which are physiologically tolerated, especially if used on humans and/or mammals. Examples of physiologically tolerated salts of particular acids are salts of hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid, citric acid, glutamic acid, 1,1-dioxo-1,2-dihydro-6-benzo[d]isothiazol-3-one (saccharin acid), monomethylsebacic acid, 5-oxo-proline, hexane-1-sulfonic acid, nicotinic acid, 2-,3- or 4-aminobenzoic acid, 2,4,6-trimethyl-benzoic acid, alpha-lipoic acid, acetylglycine, acetylsalicylic acid, hippuric acid and/or aspartic acid. Examples of physiologically tolerated salts of particular bases are salts of alkali and alkaline earth metals and/or with {NHₓR₄₋ₓ]⁺-ions, wherein x is 0, 1, 2, 3 or 4 and R represents a linear or branched C₁₋₄ alkyl radical.

The active substance according to component (B) and/or the active substance according to component (A) of the inventive active substance combination may each also be present in form of mixture of two or more different salts.

If an active substance according to component (A) is basic and an active substance according to component (B) is acidic group or vice versa, both components may at least partially form a salt with one another. These salts may be prepared according to conventional methods well known to those skilled in the art, e.g. by dissolution of both components in a suitable solvent and subsequent evaporation of the solvent.

Thus, in another preferred embodiment of the present invention component (A) and component (B) are at least partially present in form of a salt formed between these two components.

The inventive active substance combination is suitable for the administration to humans, including infants, children and grown-ups, as well as animals.

Preferably the total amount of the active substance(s) according to component (A), calculated as the free compound(s), to be administered to the patient in a 24 hours period does not exceed 800 mg.
The total amount of the active substance(s) according to component (B), calculated as the free compound(s), to be administered to the patient in a 24 hours period does not exceed 200 mg.
Preferably the inventive active substance combination comprises components (A) and (B) in the above defined molar ratios and within the afore given limits for the maximum dosis to be administered per day.

Pharmaceutically active substances, particularly opioids, may be the subject of abuse. For example, a certain dose of an opioid active substance is usually more potent when administered parenterally, particularly intravenously, compared to the same dose being administered orally. Consequently, a common mode of abuse for an oral pharmaceutical formulation comprising an opioid active substance includes the extraction of the opioid from the formulation with subsequent intravenous injection.

Thus, in another preferred embodiment of the present invention the active substance combination further comprises as component (C) one or more agents that are suitable to reduce or even prevent abuse of the active substances of component (A) and/or component (B).

If such anti-abuse agents are present in the inventive active substance combination, they are included in such a form that they are either not liberated at all or in such a way that they do not develop their anti-abuse effect if the active substance combination is administered to the patient according to its intended route of administration.

However, if the inventive active substance combination or - after separation - one of its components alone is administered via a route other than the intended route of administration, said anti-abuse agent will exert its effect and therefore reduce or even prevent abuse.

Agents that are particularly suitable for the reduction or prevention of opioid abuse are, for example, opioid antagonists, which have little or no effect if taken orally, but which will block the effect of the opioid if administered parenterally together with the opioid after extraction.
Suitable opioid antagonists may preferably be selected from the group consisting of levallorphan, naloxone, naltrexone and corresponding physiologically acceptable salts thereof.

Other anti-abuse agents include aversive agents such as bittering agents, irritants, emetics and/or nauseants as well as gelling agents.

The kinds and amounts of the anti-abuse agents used as component (C) in the inventive active substance combination as well as their mode of formulation together with components (A) and/or (B) depend on the kind of abuse that is to be reduced or prevented, e.g. parenteral, intranasal or oral misuse. Different modes of formulations and/or different anti-abuse agents from the same class or from different classes may be used to reduce or eliminate more than one kind of abuse, e.g. the inventive active substance combination may comprise one agent suitable for the reduction or prevention of parenteral abuse and one agent suitable for the reduction or prevention of nasal abuse.

Suitable opioid antagonists according to component (C) of the inventive substance combination as well as methods for their preparation are well known to those skilled in the art, e.g. from E. Friderichs, T. Christoph and H. Buschmann, "Analgesics and Antipyretics", Ullmann's Encyclopedia of Industrial Chemistry, Sixth Edition, Wiley-VCH Verlag GmbH, Weinheim 2000, pages 45-47.

Opioid antagonists as well as other anti-abuse agents suitable for reduction or prevention of different ways of abuse of active substances, suitable amounts and methods for their incorporation into pharmaceutical formulations are also known to those skilled in the art from EP 1 201 233, WO03/013476 and WO 99/32120.

The respective parts of the descriptions of the afore mentioned publications are hereby incorporated by references and form part of the present disclosure.

In another aspect the present invention relates to a medicament comprising an inventive active substance combination and optionally at least one further active substance and/or optionally at least one auxiliary substance.

Preferably the inventive medicament is suitable for the treatment of pain, particularly for the treatment of pain selected from the group consisting of neuropathic pain, acute pain, chronic pain, post-operative pain, chronic lower back pain, cluster headaches, herpes neuralgia, phantom limb pain, central pain, dental pain, resistant pain, visceral pain, surgical pain, bone injury pain, pain during labor and delivery, pain resulting from burns, pain resulting from sunburns, post partum pains, migraine, angina pain, genitourinary tract-related pain, pain from cystitis and nociceptive pain. Also preferably the inventive medicament is suitable for the prophylaxis and/or treatment of urinary incontinence. Furthermore, the inventive medicament is also suitable for the prophylaxis and/or treatment of neurogenic inflammation.

Those skilled in the art understand that the components (A) and (B) of the active substance combination according to the present invention may be administered simultaneously or sequentially to one another, whereby in each case components (A) and (B) may be administerd via the same or different administration pathways, e.g. orally or parenterally, preferably both components (A) and (B) are administered simultaneously in one and the same administration form.

Another aspect of the present invention relates to the use of an inventive pharmacologically active substance combination for the preparation of a medicament for the treatment of pain, preferably for the treatment of pain selected from the group consisting of neuropathic pain, acute pain, chronic pain, post-operative pain, chronic lower back pain, cluster headaches, herpes neuralgia, phantom limb pain, central pain, dental pain, resistant pain, visceral pain, surgical pain, bone injury pain, pain during labor and delivery, pain resulting from burns, pain resulting from sunburns, post partum pains, migraine, angina pain, genitourinary tract-related pain, pain from cystitis and nociceptive pain. A further aspect of the present invention is the use of an inventive active substance combination for the preparation of a medicament for the prophylaxis and/or treatment of urinary incontinence. Yet another aspect of the present invention is the use of an inventive active substance combination for the preparation of a medicament for the prophylaxis and/or treatment of neurogenic inflammation.

A further aspect of the present invention relates to pharmaceutical formulations in different pharmaceutical forms comprising an inventive active substance combination and optionally at least one further active substance and/or optionally at least one auxiliary substance.

Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.

Inventive pharmaceutical formulation for oral administration are preferably selected from the group consisting of tablets, drageés, capsules, drops, gels, juices, sirups, solutions and suspensions.

The pharmaceutical formulation of the present invention for oral administration may also be in the form of multiparticulates, preferably pellets or granules, optionally compressed into a tablet, filled into a capsule or suspended in a suitable liquid. Suitable liquids are known to those skilled in the art.

The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.

In one embodiment of the present invention the pharmaceutical formulation comprises one or both of the components (A) and (B) at least partially in a sustained-release form. Preferably the inventive pharmaceutical formulation comprises component (B) at least partially in a sustained-release form.

By incorporating one or both of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D®), copolymers of ethyl acrylate, methyl methacrylate and trimethylammonium ethyl methacrylate-chloride with a monomer molar ratio of 1:2:0.1 (Eudragit RS®), copolymers of ethyl acrylate, methyl methacrylate and trimethylammonium ethyl methacrylate-chloride with a monomer molar ratio of 1:2:0.2 (Eudragit RL®), or a mixture of at least two of the above-mentioned copolymers. These coating materials are commercially available as 30 wt.% aqueous latex dispersions, i.e. as Eudragit RS30D®, Eudragit NE30D® or Eudragit RL30D®, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat® or Surelease®.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet® (acetylated mono- and diglycerides, C₂₃H₄₄O₅ to C₂₅H₄₇O₇), medium-chain triglycerides (Miglyol®), oleic acid or mixtures of at least two of said plasticizers. Aqueous dispersions of Eudragit RS® and optionally Eudragit RL® preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30D-55®), methacrylic acid/methyl acrylate/methyl methacrylate copolymers with a monomer molar ratio of 7:3:1 (Eudragit FS®), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D® and/or Eudragit RL® and/or Eudragit RS®.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

It has surprisingly been found that in the active substance combination of the present invention the pharmacological efficacy of the opioid component is enhanced by their administration in combination with one or more substituted carbinol compounds of general formula I given above. As a result of this synergistic effect, the dose of the opioid may be reduced and fewer, less pronounced to none undesired side effects occur and the risk of tolerance development is reduced, while the analgesic efficacy is at least maintained.
In addition to this, the withdrawal symptoms resulting from the administration of such an opioid analgesic component are reduced or entirely suppressed, even if the amount of administered opioid analgesic is not reduced compared to the administration of an opioid analgesic alone.

### Pharmacological Methods:

### A. Hot plate test in mice:

The analgesic activity of the inventive active substance combination is determined in male Swiss mice (weight 20-25 g, Harlan Iberica, S. Feliu de Codinas, Barcelona, Spain) as described in the publication of G. Woolfe and A. D. MacDonald, J. Pharm. Exp. Ther. 1944, 80, pages 300-307. The respective description of this publication is incorporated by reference and forms part of the present disclosure.

According to this test the mice are put onto a plate, which is heated to 55 °C and the time is determined until the mice show signs of pain such as vigorous and repeated licking of the paws, jumping or pulling back the paws. The mice are kept on the hot plate for no longer than 40 seconds in order to avoid the development of cutaneous lesions. At first the untreated mice are subjected to the hot-plate test to determine a baseline for their pain induced behaviour. After 10 minutes the vehicle, the active substances and the inventive active substance combination to be tested are administered to different groups of mice. 0.5 hours, 1 hour and 2 hours after the administration the animals are put onto the hot plate and the time is measured until they show signs of pain. The analgesic efficacy of the active substances or active substance combination is calculated on the basis of the values obtained for the comparison group of mice which is only administered the vehicle.

### B. Determination of withdrawal symptoms

The effect of the inventive active substance combination on withdrawal symptoms after treatment with an opoid is determined according to the publication of J.K. Saelens et al. Int. Pharmacodyn. 1971, 190, pages 213-218 in male Swiss albino mice (weight 20-25 g, Harlan Iberica, S. Feliu de Codinas, Barcelona, Spain).

Opioid dependency is delevoped in the mice by intraperitoneal administration of the opioid in a suitable dose known to those skilled in the art, e.g. 5 mg/kg/day for 4 consecutive days for morphine. Withdrawal symptoms are then induced by the intravenous administration of a suitable opioid antagonist in a dose known to those skilled in the art, for example, 2 mg/kg naloxone in case of morphine, 30 minutes after the administration of the final dose of the opioid is completed.

In the 30 minute perioid following the naloxone administration the mice show typical withdrawal symptoms, namely jumps and shakes (of the wet dog shake type), which are counted and registered.

The active substance comination is also administered to the mice for 4 consecutive days. After the administration of 2 mg/kg naloxone 30 minutes after the administration of the final dose of the active substance combination has been completed, the mice are closely watched for withdrawal symptoms, namely jumps and shakes (of the wet dog shake type), which are then counted and registered.

The present invention is illustrated below with the aid of examples. These illustrations are given solely by way of example and do not limit the general spirit of the present invention.

### Examples:

### A. Hot plate test in mice:

The analgesic efficacy of an inventive active substance combination comprising as component (A) the compound (±)-5-[α-[2-(Dimethylamino)ethoxy]benzyl]-1-methyl-1H-pyrazole citrate (hereinafter Cizolirtine Citrate) and as component (B) Morphine in mice has been determined as described above and compared to the administration of vehicle, Cizolirtine Citrate and Morphine alone.

The active substance combination as well as the comparison substances, their mode of administration and the respective amounts are given in the following table A together with determined activities.

**Table A:**

| Preparation administered | Dosis (mg/kg) | Mode of administration | % Analgesic activity | | |
|---|---|---|---|---|---|
| | | | 0.5 hours¹ | 1 hour¹ | 2 hours¹ |
| vehicle² | --- | s.c. | 7.8 | 9.6 | 6.6 |
| Cizolirtine³ | 20 | i.p. | 25.8 | 17.1 | 23.4 |
| Morphine | 5 | s.c. | 48.6 | 34.8 | 15.8 |
| Cizorlitine³ + Morphine | 20 + 5 | i.p. s.c. | 88.8 | 71.5 | 30.7 |

| | | | | | |
|---|---|---|---|---|---|
| 1 time after administration | | | | | |
| 2: 5 % by weight arabic gum in water for injection purposes | | | | | |
| 3: as Citrate salt s.c.: subcutaneous i.p.: intraperitoneal | | | | | |

As can be seen from table A the inventive active substance combination shows a synergistic effect.

### B: Determination of withdrawal symptoms

The influence of the active substance combination on withdrawal symptons has been determined as described above. The results are given in Table B:

**Table B:**

| Preparation administered | Dosis (mg/kg/day) over 4 days | mode of administration | Number of jumps | Number of shakes |
|---|---|---|---|---|
| vehicle¹ | --- | i.p. | 0 | 0 |
| Morphine | 5 | s.c. | 6.6 | 16.3 |
| Cizorlitine² + Morphine | 40 + 5 | i.p. s.c. | 3.1 | 3.4 |

| | | | | |
|---|---|---|---|---|
| 1: 5 % by weight arabic gum in water for injection purposes | | | | |
| 2: as Citrate salt i.p.: intraperitoneal s.c. subcutaneous | | | | |

As can be seen from the values given in table B the withdrawal symptoms usually associated with the administration of opoids (here morphine) are significantly reduced by its co-administration with a substituted carbinol compound - component (A) - Cizolirtin Citrate, even if the administered amount of the opioid is not reduced.

## Claims

1. Active substance combination comprising
(A) at least one substituted carbinol compound of general formula I, 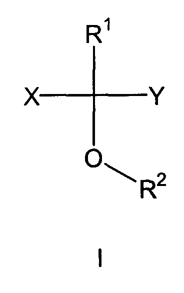 wherein
R¹ represents a hydrogen atom, a linear or branched alkyl radical, a linear or branched alkenyl radical, an optionally at least mono-substituted cycloaliphatic radical, which may contain at least one nitrogen atom as ring member, or a phenyl radical,
R² represents a hydrogen atom, an optionally at least one nitrogen atom as ring member containing cycloaliphatic radical, which may be at least mono-substituted by a linear or branched alkyl radical and/or which may be bound via a linear or branched alkylene group, an NR³R⁴-moiety, which is bound via a linear or branched alkylene group, or an NR⁵R⁶-moiety, which is bound via a linear or branched alkylene group,
R³ and R⁴, identical or different, represent a linear or branched alkyl radical or an unsubstituted benzyl radical,
R⁵ and R⁶ together with the bridging nitrogen atom represent a saturated, unsubstituted, optionally at least one further heteroatom as ring member containing heterocyclic radical,
X represents an optionally at least mono-substituted phenyl radical or an optionally at least mono-substituted thienyl radical, wherein in each case the substituents may be independently selected from the group consisting of a linear or branched alkyl radical, a linear or branched alkoxy group, a linear or branched alkyl radical, which is at least partially halogenated and a halogen atom,
Y represents a heteroaryl radical, which contains one or more nitrogen atoms as ring members and which is unsubstituted or at least mono-substituted by one or more substitutents independently from one another selected from the group consisting of a halogen atom, a linear or branched alkyl radical, a benzyl radical, a ciano group bound via a linear or branched C₁₋₄-alkylene group, a carboxy group bound via a linear or branched C₁₋₄-alkylene group, a methoxy carbonyl group bound via a linear or branched C₁₋₄-alkylene group, a hydroxy group bound via a linear or branched C₁₋₄-alkylene group, an amino group bound via a linear or branched C₁₋₄-alkylene group, a (C₁₋₄) dialkylamino group bound via a linear or branched C₁₋₄-alkylene group, and a cycloaliphatic radical, which contains at least one nitrogen atom as ring member and which is bound via a linear or branched C₁₋₄-alkylene group, or Y represents an unsubstituted heteroaryl radical, which contains two nitrogen atoms as ring members and which is condensed with a saturated, one methyl-substituted nitrogen atom as ring member containing cycloaliphatic group,
optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate, and
(B) at least one opioid.

2. Active substance combination according to claim 1, **characterized in that** R¹ represents a hydrogen atom, a linear or branched C₁₋₄ alkyl radical, a linear or branched C₂₋₄ alkenyl radical, a 5- or 6-membered cycloaliphatic radical, which may contain at least one nitrogen atom as ring member and/or which may be at least mono-substituted by a linear or branched C₁₋₄ alkyl radical, or a phenyl radical, preferably a hydrogen atom, a linear or branched C₁₋₄ alkyl radical, a vinyl group, a cyclohexyl radical, an N-Methyl-piperidyl radical or a phenyl radical.

3. Active substance combination according to claim 1 or 2, **characterized in that** R² represents a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³R⁴-moiety, which is bound via a linear or branched C₁₋₄ alkylene group, or a NR⁵R⁶-moiety, which is bound via a linear or branched C₁₋₄ alkylene group, preferably a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³R⁴-moiety, which is bound via a linear or branched C₂₋₃ alkylene group, or a NR⁵R⁶-moiety, which is bound via a linear or branched C₂₋₃ alkylene group.

4. Active substance combination according to one or more of claims 1-3, **characterized in that** R³ and R⁴, identical or different, independently from one another represent a linear or branched C₁₋₄ alkyl radical or an unsubstituted benzyl radical, preferably a linear or branched C₁₋₄ alkyl radical.

5. Active substance combination according to one or more of claims 1-4, **characterized in that** R⁵ and R⁶ together with the bridging nitrogen atom represent a saturated, unsubstituted, optionally at least one oxygen atom as ring member containing, 5- or 6-membered heterocyclic radical.

6. Active substance combination according to one or more of claims 1-5, **characterized in that** X represents an optionally at least mono-substituted phenyl radical or an optionally at least mono-substituted thienyl radical, wherein in each case the substituents may be independently selected from the group consisting of a linear or branched C₁₋₄ alkyl radical, a linear or branched C₁₋₄ alkoxy radical, a linear or branched C₁₋₄ alkyl radical, which is at least partially fluorinated, a fluorine atom, a chlorine atom and a bromine atom, preferably represents an optionally at least mono-substituted phenyl radical or an optionally at least mono-substituted thienyl radical, wherein in each case the substituents may be independently selected from the group consisting of a methyl radical, a methoxy radical, a trifluoromethyl radical, a fluorine atom, a chlorine atom and a bromine atom.

7. Active substance combination according to one or more of claims 1-6, **characterized in that** Y represents an azole radical selected from the group consisting of
a) a pyrazole of the general formula (a): 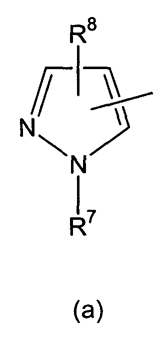 in which R⁷ represents a linear or branched C₁₋₁₂ alkyl radical, a benzyl radical or a radical of the type: 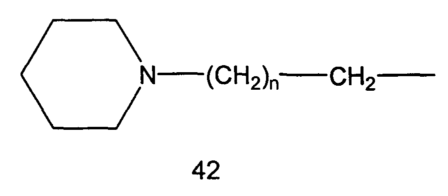 in which n = 1 or 2, and
R⁸ represents a hydrogen atom, a methyl radical or a halogen atom, preferably a hydrogen atom, a methyl radical, a bromine atom or a chlorine atom,
b) an imidazole of the general formula 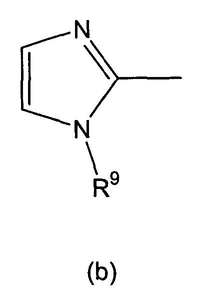 in which R⁹ represents a hydrogen atom, a C₁₋₁₂ alkyl radical, a benzyl radical, or a radical of the general formula (b1):
R¹⁰-(CH₂)ₙ- (b1)
in which n is 2, 3 or 4 and R¹⁰ represents a piperidinyl radical, a phenyl radical, a cyano group, a hydroxyl radical, a carboxy radical, an amino group, a dimethylamino group, or a methyl ester (CH₃-O-C(=O)-) group,
and
(c) an imidazole of the following formula:

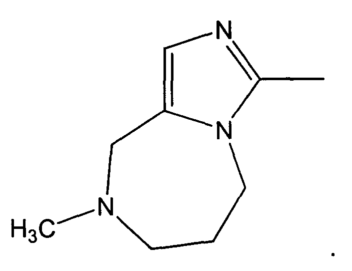

8. Active substance combination according to one or more of claims 1-7, **characterized in that** as component (A) at least one carbinol compound of general formula I 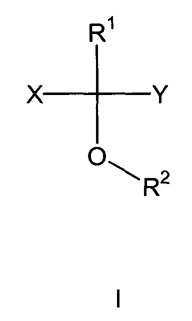 is present, wherein
R¹ represents a hydrogen atom, a methyl radical, an ethyl radical, an n-propyl radical, an iso-propyl radical, a sec-butyl radical, a tert-butyl radical, an n-butyl radical, a vinyl radical, a cyclohexyl radical, an N-methyl-piperidinyl group, or a phenyl group,
R² represents a hydrogen atom, a dimethylaminoethyl group, a pyrrolidinylethyl group, a piperidinylethyl group, a methyl-benzyl-aminoethyl group, a morpholinylethyl group, a diisopropylaminoethyl group, a dimethylaminopropyl group, a piperidinylpropyl group, a pyrrolidinylpropyl group, a morpholinylpropyl group, an N-methyl-2-piperidyl group, an N-ethyl-2-piperidyl group, an N-propyl-2-piperidyl group, an N-methyl-2-pyrrolidinyl group, an N-ethyl-2-pyrrolidinyl group, an N-propyl-2-pyrrolidinyl group, or a 2-dimethylaminoethyl-1-methyl group,
X represents a phenyl radical, a 2-methyl-phenyl radical, a 3-methyl-phenyl radical, a 4-methyl phenyl radical, a 2-chloro-phenyl radical, a 3-chloro-phenyl radical, a 4-chloro-phenyl radical, a 2-fluoro-phenyl radical, a 3-fluoro-phenyl radical, a 4-fluoro-phenyl radical, a 2-trifluoromethyl-phenyl radical, a 3-trifluoromethyl-phenyl radical, a 4-trifluoromethyl-phenyl radical, a 2-methoxy-phenyl radical, a 3-methoxy-phenyl radical, a 4-methoxy-phenyl radical, a 3,4,5-tris-methoxy phenyl radical, a 3,4-dichloro-phenyl radical, a 2,4-dichloro-phenylradical, a thien-2-yl radical, a thien-3-yl radical, a 3-methyl-thien-2-yl radical, a 5-methyl-thien-2-yl-radical, a 5-bromo-thien-2-yl radical or a 4-bromothien-2-yl-radical,
Y represents an azole radical selected from the group consisting of
a) a pyrazole of the general formula (a): 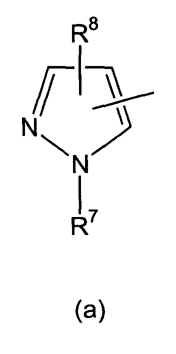 in which
R⁷ represents a methyl radical, an ethyl radical, an n-propyl radical, an iso-propyl radical, an n-butyl radical, a sec-butyl radical or a tert-butyl radical,
R⁸ represents a hydrogen atom, a methyl radical, a bromine atom or a chlorine atom,
b) an imidazole of the general formula 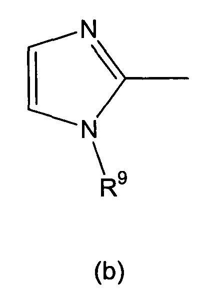 in which R⁹ represents a hydrogen atom, a methyl radical, an ethyl radical, an n-propyl radical, an iso-butyl radical, an n-butyl radical, a sec-butyl radical a tert-butyl radical, an n-pentyl radical, an n-hexyl radical, an n-heptyl radical, an n-octyl radical, an n-nonyl radical, an n-decyl radical, an n-undecyl radical an n-dodecyl radical, a benzyl radical, or a radical of the general formula (b1):
R¹⁰-(CH₂)ₙ- (b1)
in which n is 2, 3 or 4 and R¹⁰ represents a piperidinyl radical, a phenyl radical, a cyano group, a hydroxyl radical, a carboxy radical, an amino group, a dimethylamino group, or a methyl ester group,
and
(c) an imidazole of the following formula: 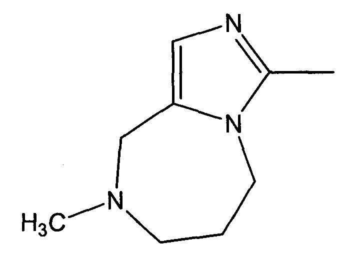 optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

9. Active substance combination according to one or more of claims 1-8, **characterised in that** as component (A) one or more compounds selected from the group consisting of
[1]2-{α-[2-(dimethylamino)ethoxy]benzyl}-1-methyl-1*H*-imidazole,
[2]2-{4-chloro-α-[2-(dimethylamino)ethoxy]-α-methylbenzyl}-1-methyl-1*H*-imidazole,
[3]2-{4-chloro-α-[2-(dimethylamino)ethoxy]benzyl}-1-methyl-1*H*-imidazole,
[4]2-{3-chloro-α-[2-(dimethylamino)ethoxy]benzyl}-1-methyl-1*H*-imidazole,
[5]2-{4-chloro-α-[2-(dimethylamino)ethoxy]-α-methylbenzyl}-1-methyl-1*H*-imidazole,
[6]2-{4-fluoro-α-[2-(dimethylamino)ethoxy]-α-methylbenzyl}-1-methyl-1*H*-imidazole,
[7]2-{α-[2-(dimethylamino)ethoxy]-α-methyl-3-(trifluoromethyl)benzyl}-1-methyl-1*H*-imidazole,
[8]2-{3-chloro-α-[2-(dimethylamino)ethoxy]-α-methylbenzyl}-1-methyl-1*H*-imidazole,
[9]2-{3-chloro-α-[2-(dimethylamino)ethoxy]-α-propylbenzyl}-1-methyl-1*H*-imidazole,
[10] 1-butyl-2-{4-chloro-α-[2-(dimethylamino)ethoxy]-α-methylbenzyl}-1*H*-imidazole,
[11] 2-{α-[2-(dimethylamino)ethoxy]-α-methyl-4-methoxybenzyl}-1-methyl-1*H*-imidazole,
[12] 2-{3-chloro-α-methyl-α-[2-(N-pyrrolidinyl)ethoxy]benzyl}-1-methyl-1*H*-imidazole,
[13] 2-{α-[2-(dimethylamino)ethoxy]-α-propyl-3,4,5-trimethoxybenzyl}-1-dodecyl-1*H*-imidazole,
[14] 1-butyl-2-{α-[2-(dimethylamino)ethoxy]-4-(trifluoromethyl)benzyl}-1*H*-imidazole,
[15] 1-methyl-2-{α-methyl-α-[2-(N-piperidyl)ethoxy]-3-(trifluoromethyl)benzyl}-1*H*-imidazole,
[16] 2-{α-cyclohexyl-3,4-dichloro-α-[2-(dimethylamino)ethoxy]benzyl}-1-methyl-1*H*-imidazole,
[17] 2-{3,4-dichloro-α-[2-(dimethylamino)ethoxy]-α-propylbenzyl}-1-methyl-1*H*-imidazole,
[18] 2-{3,4-dichloro-α-[2-(dimethylamino)ethoxy]-α-methylbenzyl}-1-methyl-1*H*-imidazole,
[19] 2-{3,4-dichloro-α-[2-(dimethylamino)ethoxy]benzyl}-1-methyl-1*H*-imidazole,
[20] 2-{4-chloro-α-[2-(dimethylamino)ethoxy]-α-methylbenzyl}-1-[2-(N-piperidyl)ethyl]-1*H*-imidazole,
[21] 2-{4-chloro-α-[2-(dimethylamino)ethoxy]-α-methylbenzyl}-1-[2-(N-piperidyl)propyl]-1*H*-imidazole,
[22] 1-(3-cyanopropyl)-2-{4-chloro-α-[2-(dimethylamino)ethoxy]benzyl}-1*H*-imidazole,
[23] 2-{4-chloro-α-[2-(dimethylamino)ethoxy]-α-(N-methyl-4-piperidyl)benzyl}-1-methyl-1*H*-imidazole,
[24] 1-benzyl-2-{α-[2-(N-benzyl-N-methylamino)ethoxy]-4-chlorobenzyl}-1*H*-imidazole,
[25] 2-{4-chloro-α-[2-(dimethylamino)ethoxy]-α-methylbenzyl}-7-methyl-6,7,8,9-tetrahydro-1*H*-imidazole[1,5-a][1,4]diazepine,
[26] 2-{4-chloro-α-[2-(dimethylamino)ethoxy]benzyl}-7-methyl-6,7,8,9-tetrahydro-1*H*-imidazole[1,5-a][1,4]diazepine,
[27] 1-butyl-5-{α-[2-(dimethylamino)ethoxy]benzyl}-1*H*-pyrazole,
[28] 5-{α-(4-chlorophenyl)-α-[2-(dimethylamino)ethoxy]benzyl}-1-methyl-1*H*-pyrazole,
[29] 1-butyl-5-{α-[2-(dimethylamino)ethoxy]-3,4,5-trimethoxybenzyl}-1*H*-pyrazole,
[30] 1 -butyl-5-{4-chloro-α-[2-(dimethylamino)ethoxy]-α-methylbenzyl}-1*H*-pyrazole,
[31] 5-{α-[2-(dimethylamino)ethoxy]benzyl}-1-methyl-1*H*-pyrazole,
[32] 5-{α-[2-(dimethylamino)ethoxy]-α-methylbenzyl}-1-methyl-1*H*-pyrazole,
[33] 5-{α-[2-(dimethylamino)ethoxy]-3,4,5-trimethoxybenzyl}-1-methyl-1*H*-pyrazole,
[34] 1-methyl-5-{α-[2-(N-pyrrolidinyl)ethoxy]benzyl}-1*H*-pyrazole,
[35] 1-methyl-5-{α-[2-(N-morpholinyl)ethoxy]benzyl}-1*H*-pyrazole,
[36] 5-{α-[2-(dimethylamino)ethoxy]-α-methyl-3,4,5-trimethoxybenzyl}-1-methyl-1*H-*pyrazole,
[37] 4-bromo-5-{α-[2-(dimethylamino)ethoxy]benzyl}-1-methyl-1*H*-pyrazole,
[38] 1,3-dimethyl-5-{α-[2-(dimethylamino)ethoxy]-α-methylbenzyl}-1*H*-pyrazole,
[39] 1,3-dimethyl-5-{α-[2-(dimethylamino)ethoxy]benzyl}-1*H*-pyrazole,
[40] 5-{α-[2-(dimethylamino)ethoxy]-2-methylbenzyl}-1-methyl-1*H*-pyrazole,
[41] 4-chloro-5-(4-chloro-α-[2-(dimethylamino)ethoxy]benzyl)-1-methyl-1*H*-pyrazole,
[42] 5-{4-chloro-α-[2-(dimethylamino)ethoxy]benzyl}-1-methyl-1*H*-pyrazole,
[43] 5-{3-chloro-α-[2-(dimethylamino)ethoxy]benzyl}-1-methyl-1*H*-pyrazole,
[44] 5-{α-[2-(dimethylamino)ethoxy]-4-methylbenzyl}-1-methyl-1*H*-pyrazole,
[45] 5-{2-chloro-α-[2-(dimethylamino)ethoxylbenzyl}-1-methyl-1*H*-pyrazole,
[46] 1-methyl-5-{α-[2-(N-piperidyl)ethoxy]benzyl}-1*H*-pyrazole,
[47] 1-methyl-5-{α-[2-(N-propyl-2-piperidyl)ethoxy]benzyl}-1*H*-pyrazole,
[48] 5-{α-[2-(N-ethyl-2-piperidyl)ethoxy]benzyl}-1-methyl-1*H*-pyrazole,
[49] 1-methyl-5-{α-[2-(N-methyl-2-pyrrolidinyl)ethoxy]benzyl}-1*H*-pyrazole,
[50] 5-{α-[2-(diisopropylamino)ethoxy]benzyl}-1-methyl-1*H*-pyrazole,
[51] 1-methyl-5-{α-[2-(N-methyl-2-piperidyl)ethoxy]benzyl}-1*H*-pyrazole,
[52] 2-{4-chloro-α-[3-(dimethylamino)propoxy]-α-methylbenzyl}-1-methyl-1*H*-imidazole,
[53] 2-{3-chloro-α-[3-(dimethylamino)propoxy]benzyl}-1-methyl-1*H*-imidazole,
[54] 2-{4-chloro-α-[3-(dimethylamino)propoxy]-α-ethylbenzyl}-1-methyl-1*H*-imidazole,
[55] 2-{α-butyl-3-chloro-α-[3-(dimethylamino)propoxy]benzyl}-1-methyl-1*H*-imidazole,
[56] 2-{α-cyclohexyl-4-chloro-α-[3-(dimethylamino)propoxy]benzyl}-1-methyl-1*H*-imidazole,
[57] 2-{α-[3-(dimethylamino)propoxy]-4-fluoro-α-methylbenzyl}-1-methyl-1*H*-imidazole,
[58] 2-{α-[3-(dimethylamino)propoxy]-α-methyl-3-(trifluoromethyl)benzyl}-1-methyl-1*H*-imidazole,
[59] 2-{2-chloro-α-[3-(dimethylamino)propoxy]-α-methylbenzyl}-1-methyl-1*H*-imidazole,
[60] 2-{3-chloro-α-[3-(dimethylamino)propoxy]-α-methylbenzyl}-1-methyl-1*H*-imidazole,
[61] 2-{α-[3-(dimethylamino)propoxy]-α-methyl-3,4,5-trimethoxybenzyl}-1-methyl-1*H*-imidazole,
[62] 2-{α-(3-(dimethylamino)propoxy]-α-methyl-4-methoxybenzyl}-1-methyl-1*H*-imidazole,
[63] 2-{4-chloro-α-[3-(dimethylamino)propoxy]benzyl}-1-methyl-1*H*-imidazole,
[64] 2-{α-[3-(dimethylamino)propoxy]-3,4,5-trimethoxybenzyl}-1-methyl-1*H*-imidazole,
[65] 2-{α-[3-(dimethylamino)propoxy]-α-methyl-4-(trifluoromethyl)benzyl}-1-methyl-1*H*-imidazole,
[66] 2-{α-[3-(dimethylamino)propoxy]-3-(trifluoromethyl)benzyl}-1-methyl-1*H*-imidazole,
[67] 2-{α-[3-(dimethylamino)propoxy]-4-(trifluoromethyl)benzyl}-1-methyl-1*H*-imidazole,
[68] 2-{α-[3-(dimethylamino)propoxy]-4-methoxybenzyl}-1-methyl-1*H*-imidazole,
[69] 2-{α-butyl-α-[3-(dimethylamino)propoxy]-3-(trifluoromethyl)benzyl}-1-methyl-1*H*-imidazole,
[70] 1-butyl-2-{4-chloro-α-[3-(dimethylamino)propoxy]-α-methylbenzyl}-1*H*-imidazole,
[71] 1-butyl-2-{α-butyl-α-[3-(dimethylamino)propoxy]-3,4,5-trimethoxybenzyl}-1*H*-imidazole,
[72] 1-butyl-2-{α-butyl-2-chloro-α-[3-(dimethylamino)propoxy]benzyl}-1*H*-imidazole,
[73] 1-butyl-2-{α-butyl-2,4-dichloro-α-[3-(dimethylamino)propoxy]benzyl}-1*H*-imidazole,
[74] 1-butyl-2-{α-[3-(dimethylamino)propoxy]-4-(trifluoromethyl)benzyl}-1*H*-imidazole,
[75] 2-{4-chloro-α-[3-(N-piperidyl)propoxy]benzyl}-1-methyl-1*H*-imidazole,
[76] 1-methyl-2-{α-methyl-α-[3-(N-piperidyl)propoxy]-4-(trifluoromethyl)benzyl}-1*H*-imidazole,
[77] 2-{α-butyl-2-chloro-α-[3-(dimethylamino)propoxy]benzyl}-1-methyl-1*H*-imidazole,
[78] 2-{α-butyl-3,4-dichloro-α-[3-(dimethylamino)propoxy]benzyl}-1-methyl-1*H*-imidazole,
[79] 2-{3,4-dichloro-α-[3-(dimethylamino)propoxy]-α-methylbenzyl}-1-methyl-1*H*-imidazole,
[80] 2-{3,4-dichloro-α-[3-(dimethylamino)propoxy]benzyl}-1-methyl-1*H*-imidazole,
[81] 2-{α-cyclohexyl-3,4-dichloro-α-[3-(dimethylamino)propoxy]benzyl}-1-methyl-1*H*-imidazole,
[82] 2-{4-chloro-α-[3-(dimethylamino)propoxy]-α-methylbenzyl}-α-[2-(N-piperidyl) ethyl]-1*H*-imidazole,
[83] 2-{4-chloro-α-[3-(dimethylamino)propoxy]-α-methylbenzyl}-1-[2-(N-piperidyl) propyl]-1*H*-imidazole,
[84] 2-{4-chloro-α-[3-(dimethylamino)propoxy]-α-(N-methyl-4-piperidyl)benzyl}-1-methyl-1*H*-imidazole,
[85] 1-butyl-5-{α-[3-(dimethylamino)propoxy]benzyl}-1*H*-pyrazole,
[86] 1-butyl-5-{4-chloro-α-[3-(dimethylamino)propoxy]-α-methylbenzyl}-1*H*-pyrazole,
[87] 5-{α-[3-(dimethylamino)propoxy]benzyl}-1-methyl-1*H*-pyrazole,
[88] 5-{α-[3-(dimethylamino)propoxy]-α-methylbenzyl}-1-methyl-1*H*-pyrazole,
[89] 1,3-dimethyl-5-{α-[3-(dimethylamino)propoxy]-α-methylbenzyl}-1*H*-pyrazole,
[90] 1,3-dimethyl-5-{α-[3-(dimethylamino)propoxy]benzyl}-1*H*-pyrazole,
[91] 5-{α-[3-(dimethylamino)propoxy]-2-methylbenzyl}-1-methyl-1*H*-pyrazole,
[92] 5-chloro-5-{4-chloro-α-[3-(dimethylamino)propoxy]benzyl}-1-methyl-1*H*-pyrazole,
[93] 1-methyl-5-{α-[3-(N-piperidyl)propoxy]benzyl}-1*H*-pyrazole,
[94] 1-methyl-5-{α-[3-(N-pyrrolidinyl)propoxy]benzyl}-1*H*-pyrazole,
[95] 4-{4-chloro-α-[2-(dimethylamino)ethoxy]benzyl}-1-methyl-1*H*-pyrazole,
[96] 4-{4-chloro-α-[2-(dimethylamino)ethoxy]-α-methylbenzyl}-1-methyl-1*H*-pyrazole,
[97] 4-{4-chloro-α-[2-(N-propyl-2-piperidyl)ethoxy]benzyl}-1-methyl-1*H*-pyrazole,
[98] 4-{4-chloro-α-[2-(N-methyl-2-piperidyl)ethoxy]benzyl}-1-methyl-1*H*-pyrazole,
[99] 4-{4-chloro-α-[2-(N-ethyl-2-piperidyl)ethoxy]benzyl}-1-methyl-1*H*-pyrazole,
[100] 4-{4-chloro-α-[2-(diisopropylamino)ethoxy]benzyl}-1-methyl-1*H*-pyrazole,
[101] 4-{4-chloro-α-[2-(N-methyl-2-pyrrolidinyl)ethoxy]benzyl}-1-methyl-1*H*-pyrazole,
[102] 4-{α-[3-(dimethylamino)propoxy]benzyl}-1-methyl-1*H*-pyrazole,
[103] 4-{4-chloro-α-[3-(N-morpholinyl)propoxy]benzyl}-1-methyl-1*H*-pyrazole,
[104] 4-{4-chloro-α-[3-(N-pyrrolidinyl)propoxy]benzyl}-1-methyl-1*H*-pyrazole,
[105] 2-(α-hydroxybenzyl)-1*H*-imidazole,
[106] 2-(4-chloro-α-hydroxybenzyl)-1*H*-imidazole,
[107] 2-(4-chloro-α-hydroxybenzyl)-1-methyl-1*H*-imidazole,
[108] 2-(3-chloro-α-hydroxybenzyl)-1-methyl-1*H*-imidazole,
[109] 2-(4-fluoro-α-hydroxybenzyl)-1-methyl-1*H*-imidazole,
[110] 2-[α-hydroxy-3-(trifluoromethyl)benzyl]-1-methyl-1*H*-imidazole,
[111] 2-[α-hydroxy-4-(trifluoromethyl)benzyl]-1-methyl-1*H*-imidazole,
[112] 2-(α-hydroxy-3,4,5-trimethoxybenzyl)-1-methyl-1*H*-imidazole,
[113] 2-(3,4-dichloro-α-hydroxybenzyl)-1-methyl-1*H*-imidazole,
[114] 1-butyl-2-[α-hydroxy-4-(trifluoromethyl)benzyl]-1*H*-imidazole,
[115] 1-butyl-2-(3,4-dichloro-α-hydroxybenzyl)-1*H*-imidazole,
[116] 1-butyl-2-(4-chloro-α-hydroxybenzyl)-1*H*-imidazole,
[117] 1-butyl-2-(α-hydroxy-3,4,5-trimethoxybenzyl)-1*H*-imidazole,
[118] 1-dodecyl-2-(α-hydroxy-3,4,5-trimethoxybenzyl)-1*H*-imidazole,
[119] 2-(α-butyl-3-chloro-α-hydroxybenzyl)-1-methyl-1*H*-imidazole,
[120] 2-(3-chloro-α-hydroxy-α-methylbenzyl)-1-methyl-1*H*-imidazole,
[121] 2-(4-chloro-α-hydroxy-α-methylbenzyl)-1-methyl-1*H*-imidazole,
[122] 2-[4-chloro-α-hydroxy-α-(N-methyl-4-piperidyl)benzyl]-1-methyl-1*H*-imidazole,
[123] 2-(4-chloro-α-ethyl-α-hydroxybenzyl)-1-methyl-1*H*-imidazole,
[124] 2-(α-butyl-4-chloro-α-hydroxybenzyl)-1-methyl-1*H*-imidazole,
[125] 2-(α-cyclohexyl-4-chloro-α-hydroxybenzyl)-1-methyl-1*H*-imidazole,
[126] 2-(2-chloro-(α-hydroxy-α-methylbenzyl)-1-methyl-1*H*-imidazole,
[127] 2-(α-butyl-2-chloro-α-hydroxybenzyl)-1-methyl-1*H*-imidazole,
[128] 2-[α-hydroxy-α-methyl-3-(trifluoromethyl)benzyl]-1-methyl-1*H*-imidazole,
[129] 2-[α-butyl-α-hydroxy-3-(trifluoromethyl)benzyl]-1-methyl-1*H*-imidazole,
[130] 2-[α-cyclohexyl-α-hydroxy-3-(trifluoromethyl)benzyl]-1-methyl-1*H*-imidazole,
[131] 2-[α-hydroxy-α-methyl-4-(trifluoromethyl)benzyl]-1-methyl-1*H*-imidazole,
[132] 2-(4-fluoro-α-hydroxy-α-methylbenzyl)-1-methyl-1*H*-imidazole,
[133] 2-(α-hydroxy-α-methyl-4-methoxybenzyl)-1-methyl-1*H*-imidazole,
[134] 2-(3,4-dichloro-α-hydroxy-α-methylbenzyl)-1-methyl-1*H*-imidazole,
[135] 2-(α-butyl-3,4-dichloro-α-hydroxybenzyl)-1-methyl-1*H*-imidazole,
[136] 2-(α-cyclohexyl-3,4-dichloro-α-hydroxybenzyl)-1-methyl-1*H*-imidazole,
[137] 2-(α-hydroxy-α-methyl-3,4,5-trimethoxybenzyl)-1-methyl-1*H*-imidazole,
[138] 1-butyl-2-(4-chloro-α-hydroxy-α-methylbenzyl)-1*H*-imidazole,
[139] 1-butyl-2-(α-butyl-4-chloro-α-hydroxybenzyl]-1*H*-imidazole,
[140] 1-butyl-2-[4-chloro-α-hydroxy-α-(N-methyl-4-piperidyl)benzyl]-1*H*-imidazole,
[141] 1-butyl-2-(α-butyl-α-hydroxy-3,4,5-trimethoxybenzyl)-1*H*-imidazole,
[142] 1-butyl-2-(α-butyl-2-chloro-α-hydroxybenzyl)-1*H*-imidazole,
[143] 1-butyl-2-[α-ethyl-α-hydroxy-3-(trifluoromethyl)benzyl]-1*H*-imidazole,
[144] 1-butyl-2-(α-butyl-2,4-dichloro-α-hydroxybenzyl)-1*H*-imidazole,
[145] 2-(4-chloro-α-hydroxy-α-methylbenzyl)-1-[2-(N-piperidyl)ethyl]-1*H*-imidazole,
[146] 2-(4-chloro-α-hydroxy-α-methylbenzyl)-1-(3-dimethylaminopropyl)-1*H*-imidazole,
[147] 2-(α-butyl-α-hydroxy-3,4,5-trimethoxybenzyl)-1-dodecyl-1*H*-imidazole,
[148] 1-benzyl-2-[α-butyl-α-hydroxy-3-(trifluoromethyl)benzyl]-1*H*-imidazole,
[149] 1-benzyl-2-(4-chloro-α-hydroxy-α-methylbenzyl)-1*H*-imidazole,
[150] 1-(2-cyanoethyl)-2-(4-chloro-α-hydroxybenzyl)-1*H*-imidazole,
[151] 1-(3-aminopropyl)-2-(4-chloro-α-hydroxybenzyl)-1*H*-imidazole,
[152] 3-[2-(3-chloro-α-hydroxybenzyl)-1*H*-imidazole-1-yl]propanoic acid,
[153] 2-(4-chloro-α-hydroxybenzyl)-1-(3-hydroxypropyl)-1*H*-imidazole,
[154] 3-[2-(3-chloro-α-hydroxybenzyl)-1*H*-imidazole-1-yl]methyl-propanoate,
[155] 2-(α-hydroxybenzyl)-1-(3-hydroxypropyl)-1*H*-imidazole,
[156] 2-(α-hydroxy-4-methylbenzyl)-1-(3-hydroxypropyl)-1*H*-imidazole,
[157] 2-(α-hydroxy-4-methoxybenzyl)-1-(3-hydroxypropyl)-1*H*-imidazole,
[158] 2-(3,4-dichloro-α-hydroxybenzyl)-1-(3-hydroxypropyl)-1*H*-imidazole,
[159] 3-{2-(α-hydroxybenzyl)-1*H*-imidazole-1-yll}-methyl propanoate,
[160] 2-(4-chloro-α-hydroxybenzyl)-1-(4-hydroxybutyl)-1*H*-imidazole,
[161] 1-(3-cyanopropyl)-2-(4-chloro-α-hydroxybenzyl)-1*H*-imidazole,
[162] 4-[2-(4-chloro-α-hydroxybenzyl)-1*H*-imidazole-1-yl]butanoic acid,
[163] 4-[2-(4-chloro-α-hydroxybenzyl)-1*H*-imidazole-1-yl]-methyl butanoate,
[164] 1-butyl-5-(α-hydroxybenzyl)-1*H*-pyrazole,
[165] 5-(4-chloro-α-hydroxybenzyl)-1-methyl-1*H*-pyrazole,
[166] 5-(α-hydroxy-3,4,5-trimethoxybenzyl)-1-methyl-1*H*-pyrazole,
[167] 1-butyl-5-(α-hydroxy-3,4,5-trimethoxybenzyl)-1*H*-pyrazole,
[168] 4-bromo-5-(α-hydroxybenzyl)-1-methyl-1*H*-pyrazole,
[169] 5-[α-(4-chlorophenyl)-α-hydroxybenzyl]-1-methyl-1*H*-pyrazole,
[170] 1-butyl-5-(4-chloro-α-hydroxy-α-methylbenzyl)-1*H*-pyrazole,
[171] 5-(α-hydroxy-α-methylbenzyl)-1-methyl-1*H*-pyrazole,
[172] 5-(α-hydroxy-α-methyl-3,4,5-trimethoxybenzyl)-1-methyl-1*H*-pyrazole,
[173] 1,3-dimethyl-5-(α-hydroxy-α-methylbenzyl)-1*H*-pyrazole,
[174] 1-butyl-5-(α-hydroxy-α-vinylbenzyl)-1*H*-pyrazole,
[175] 1-butyl-5-(4-chloro-α-hydroxy-α-vinylbenzyl)-1*H*-pyrazole,
[176] 4-chloro-5-(α-hydroxybenzyl)-1-methyl-1*H*-pyrazole,
[177] 5-(α-hydroxy-2-methylbenzyl)-1-methyl-1*H*-pyrazole,
[178] 5-(3-chloro-α-hydroxybenzyl)-1-methyl-1*H*-pyrazole,
[179] 5-(α-hydroxy-4-methylbenzyl)-1-methyl-1*H*-pyrazole,
[180] 5-(2-chloro-α-hydroxybenzyl)-1-methyl-1*H*-pyrazole,
[181] 5-(α-hydroxy-4-methoxybenzyl)-1-methyl-1*H*-pyrazole,
[182] 5-{α-[2-(dimethylamino)ethoxy]-2-thienylmethyl}-1-methyl-1*H*-pyrazole,
[183] 5-{α-[2-(dimethylamino)ethoxy]-2-thienylmethyl}-1-methyl-1*H*-pyrazole citrate,
[184] 5-{α-[2-(dimethylamino)ethoxy]-3-thienylmethyl}-1-methyl-1*H*-pyrazole,
[185] 2-{α-[2-(dimethylamino)ethoxy]-2-thienylmethyl}-1-methyl-1*H*-imidazole,
[186] 5-{α-[2-(dimethylamino)ethoxy]-3-methyl-2-thienylmethyl}-1-methyl-1*H*-pyrazole,
[187] 5-{α-[2-(dimethylamino)ethoxy]-5-methyl-2-thienylmethyl}-1-methyl-1*H*-pyrazole,
[188] 5-{5-bromo-α-[2-(dimethylamino)ethoxy]-2-thienylmethyl}-1-methyl-1*H*-pyrazole,
[189] 5-{4-bromo-α-[2-(dimethylamino)ethoxy]-2-thienylmethyl}-1-methyl-1*H*-pyrazole,
[190] 5-{α-[2-(dimethylamino)ethoxy]-α-methyl-2-thienylmethyl}-1-methyl-1*H*-pyrazole,
[191] 5-{α-[2-(dimethytamino)ethoxy]benzyl}-1-methyl-1*H*-pyrazole citrate,
[192] (±)-5-{α-[2-(dimethylamino)-1-(methyl)ethoxylbenzyl}-1-methyl-1*H*-pyrazole,
[193] (±)-5-{α-[2-(dimethylamino)-1-(methyl)ethoxy]benzyl}-1-methyl-1*H*-pyrazole,
[194] (+)-5-{α-[2-(dimethylamino)ethoxy]-2-thienylmethyl}-1-methyl-1*H*-pyrazole,
[195] (-)-5-{α-[2-(dimethylamino)ethoxy]-2-thienylmethyl}-1-methyl-1*H*-pyrazole,
[196] (+)-5-{α-[2-(dimethylamino)ethoxy]-2-thienylmethyl}-1-methyl-1*H*-pyrazole citrate,
[197] (-)-5-{α-[2-(dimethylamino)ethoxy]-2-thienylmethyl}-1-methyl-1*H*-pyrazole citrate,
[198] (+)-5-{α-[2-(dimethylamino)ethoxy]-2-thienylmethyl}-1-methyl-1*H*-pyrazole-D-ditoluyltartrat,
[199] (-)-5-{α-[2-(dimethylamino)ethoxy]-2-thienylmethyl}-1-methyl-1*H*-pyrazole D-ditoluyltartrat,
[200] (+)-5-{α-[2-(dimethylamino)ethoxy]benzyl}-1-methyl-1*H*-pyrazole citrate,
[201] (-)-5-{α-[2-(dimethylamino)ethoxy]benzyl}-1-methyl-1*H*-pyrazole citrate,
[202] 5-(α-hydroxy-2-thienylmethyl)-1-methyl-1*H*-pyrazole,
[203] 5-(α-hydroxy-3-methyl-2-thienylmethyl)-1-methyl-1*H*-pyrazole,
[204] 5-(α-hydroxy-5-methyl-2-thienylmethyl)-1-methyl-1*H*-pyrazole,
[205] 5-(5-bromo-α-hydroxy-2-thienylmethyl)-1-methyl-1*H*-pyrazole,
[206] 5-(4-bromo-α-hydroxy-2-thienylmethyl)-1-methyl-1*H*-pyrazole and
[207] 5-(α-hydroxy-α-methyl-2-thienylmethyl)-1-methyl-1*H*-pyrazole
are present.

10. Active substance combination according to one or more of claims 1-9, **characterized in that** as component (B) at least one opoid with weak analgesic efficacy is present.

11. Active substance combination according to claim 10, **characterized in that** the opioid with weak analgesic efficacy is selected from the group consisting of codeine, dextropropoxyphene, dihydrocodeine, diphenoxylate, ethylmorphine, loperamide, meptazinol, nalbuphine, pethidine, tilidine, tramadol, viminol and corresponding physiologically acceptable salts of these compounds.

12. Active substance combination according to claim 10 or 11, **characterized in that** the molar ratio of component (B) to component (A) is in the range of 1:1 to 1:20, preferably 1:1 to 1:10, more preferably 1:1 to 1:5.

13. Active substance combination according to one or more of claims 1-9, **characterized in that** as component (B) at least one opioid with medium to high analgesic efficacy is present.

14. Active substance combination according to claim 13, **characterized in that** the opioid with medium to high analgesic efficacy is selected from the group consisting of alfentanil, buprenorphine, butorphanol, dextromoramide, dezocine, diacetylmorphine (heroine), etorphine, fentanyl, hydrocodone, hydromorphone, ketobemidone, levomethadone, levomethadyl acetate, levorphanol, morphine, 14-Methoxymetopon, nalorphine, oxycodone, oxymorphone, pentazocine, piritramide, remifentanil, sufentanil and corresponding physiologically acceptable salts of these compounds.

15. Active substance combination according to claim 13 or 14, **characterized in that** the molar ratio of component (B) to component (A) is in the range of 1:1 to 1:400, preferably 1:1 to 1:200, more preferably 1:1 to 1:10, most preferably 1:1 to 1:5.

16. Active substance combination according to one or more of claims 1 to 15, **characterized in that** component (A) and component (B) are at least partially present as a salt formed from these components.

17. Active substance combination according to one or more of claims 1 to 16, **characterized in that** it further comprises as component (C) at least one agent, which is suitable to reduce or prevent the abuse of component (A) and/or component (B).

18. Active substance combination according to claim 17, **characterized in that** said agent(s) of component (C) is/are selected from the group consisting of opioid antagonists, aversive agents and gelling agents.

19. Active substance combination according to claim 18, **characterized in that** the opioid antagonist is selected from the group consisting of levallorphan, naloxone, naltrexone and physiologically acceptable salts thereof.

20. Medicament comprising an active substance combination according to one or more of claims 1 to 19 and optionally at least one further active substance and/or optionally at least one auxiliary substance.

21. Medicament according to claim 20 for the treatment of pain, preferably for the treatment of pain selected from the group consisting of neuropathic pain, acute pain, chronic pain, post-operative pain, chronic lower back pain, cluster headaches, herpes neuralgia, phantom limb pain, central pain, dental pain, resistant pain, visceral pain, surgical pain, bone injury pain, pain during labor and delivery, pain resulting from burns, pain resulting from sunburns, post partum pains, migraine, angina pain, genitourinary tract-related pain, pain from cystitis and nociceptive pain, or for the prophylaxis and/or treatment of urinary incontinence, or for the prophylaxis and/or treatment of neurogenic inflammation.

22. Use of an active substance combination according to one or more of claims 1 to 19 for the manufacture of a medicament for the treatment of pain.

23. Use according to claim 22, **characterized in that** the pain is selected from the group consisting of neuropathic pain, acute pain, chronic pain, post-operative pain, chronic lower back pain, cluster headaches, herpes neuralgia, phantom limb pain, central pain, dental pain, resistant pain, visceral pain, surgical pain, bone injury pain, pain during labor and delivery, pain resulting from burns, pain resulting from sunburns, post partum pains, migraine, angina pain, genitourinary tract-related pain, pain from cystitis and nociceptive pain.

24. Use of an active substance combination according to one or more of claims 1 to 19 for the manufacture of a medicament for the prophylaxis and/or treatment of urinary incontinence.

25. Use of an active substance combination according to one or more of claims 1 to 19 for the manufacture of a medicament for the prophylaxis and/or treatment of neurogenic inflammation.

26. Pharmaceutical formulation comprising an active substance combination according to one or more of claims 1 to 19 and optionally at least one further active substance and/or optionally at least one auxiliary substance.

27. Pharmaceutical formulation according to claim 26, **characterized in that** it is suitable for oral or parenteral administration, preferably for oral, intravenous, intraperitoneal, intramuscular, subcutaneous, intrathekal, rectal, transdermal, transmucosal or nasal administration.

28. Pharmaceutical formulation for oral administration according to claim 27, **characterized in that** it is in the form of a tablet, a drageé, a capsule, drops, a gel, juice, sirup, solution or suspension.

29. Pharmaceutical formulation for oral administration according to claim 27, **characterized in that** it is in form of multiparticulates, preferably pellets or granules, optionally compressed into a tablet, filled into a capsule or suspended in a suitable liquid.

30. Pharmaceutical formulation for oral administration according to claims 27-29, **characterized in that** it comprises at least one enteric coating.

31. Pharmaceutical formulation according to claim 26-30 **characterized in that** it comprises component (A) and/or component (B) at least partially in a sustained-release form.

32. Pharmaceutical formulation according to claim 31, **characterized in that** the sustained release is achieved by at least one coating or matrix comprising at least one sustained-release material.

33. Pharmaceutical formulation according to claim 32, **characterized in that** the sustained-release material is based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

34. Pharmaceutical formulation according to claim 33, **characterized in that** the water-insoluble polymer is based on an acrylic resin, which is preferably selected from the group of poly(meth)acrylates, poly(C₁₋₄)dialkylamino(C₁₋₄)alkyl (meth)acrylates and/or copolymers thereof or a mixture of at least two of the afore-mentioned polymers.

35. Pharmaceutical formulation according to claim 33, **characterized in that** the water-insoluble polymers are cellulose derivatives, preferably alkyl cellulose, particularly preferably ethyl cellulose, or cellulose esters.

36. Pharmaceutical formulation according to claim 33, **characterized in that** the wax is carnauba wax, beeswax, glycerol monostearate, glycerol monobehenate, glycerol ditripalmitostearate, microcrystalline wax or a mixture of at least two of these components.

37. Pharmaceutical formulation according to any one of claims 33-36, **characterized in that** the polymers have been used in combination with one or more plasticizers.

38. Pharmaceutical formulation according to any one of claims 31-37, **characterized in that** it comprises component (A) and/or (B) in immediate-release form as well as in sustained release form.
